# EUROPEAN PATENT SPECIFICATION

(11) **EP 4 031 106 B1**
(45) Date of publication and mention of the grant of the patent: **27.08.2025**
(21) Application number: 20775238.7
(22) Date of filing: 16.09.2020
(51) Int. Cl.: A61K 9/00, A61K 9/08, A61K 47/18, A61K 47/20, A61K 47/42, A61K 39/395

(54) **CAMPHORSULFONIC ACID AND COMBINATIONS THEREOF WITH CATIONIC EXCIPIENTS AS VISCOSITY REDUCING AGENTS IN HIGH CONCENTRATED PROTEIN FORMULATIONS**
CAMPHERSULFONSÄURE UND KOMBINATIONEN DAVON MIT KATIONISCHEN HILFSSTOFFEN ALS VISKOSITÄTSREDUZIERENDE MITTEL IN HOCHKONZENTRIERTEN PROTEINFORMULIERUNGEN
ACIDE CAMPHOSULFONIQUE ET LEURS COMBINAISONS AVEC DES EXCIPIENTS CATIONIQUES UTILISÉS EN TANT QU'AGENTS RÉDUCTEURS DE VISCOSITÉ DANS DES FORMULATIONS DE PROTÉINES CONCENTRÉES ÉLEVÉES

(30) Priority: 17.09.2019 EP 19197876; 10.12.2019 EP 19214837
(43) Date of publication of application: 27.07.2022
(73) Proprietor: Merck Patent GmbH, 64293 Darmstadt (DE)
(72) Inventor: ROSENKRANZ, Tobias, 64283 Darmstadt (DE); GUEBELI, Raphael Johannes, 64285 Darmstadt (DE); HENZLER, Tanja, 68259 Mannheim (DE); KROG, Alexandra, 55294 Bodenheim (DE); HILDEBRANDT, Christian, 64646 Heppenheim (DE)
(74) Representative: Merck Patent Association
(86) International application number: PCT/EP2020/075840
(87) International publication number: WO 2021/053001

(56) References cited:
- WO-A1-2011/139718
- WO-A1-2017/055966
- WO-A1-2019/201904
- WO-A2-2015/038818
- CA-A1- 2 943 398
- US-A1- 2019 314 505

## Description

The present invention relates to compositions of highly concentrated protein formulations showing reduced viscosity, which is induced by the addition of at least camphorsulfonic acid and at least one cationic excipient.

The contained proteins in the prepared formulations are stabilized against aggregation and denaturation and are thus sufficiently storage-stable until administration to the patient.

### State of the Art

Most biotherapeutic proteinic products in development are monoclonal antibodies (mAb) or related formats such as bi-specific antibodies or antibody fragments. The therapeutic doses of such products are often high across a broad range of clinically important indications.

However, peptide and protein molecules are larger and more complex than molecules of traditional organic and inorganic drugs (that is, they have multiple functional groups in addition to complex three-dimensional structures). The formulation of such proteins poses particular problems for the formulator. One of these problems is the increased viscosity of protein formulations, especially at high concentration.

The latter, however, is a particular problem because it is highly desirable from a patient convenience, compliance and overall healthcare cost perspective for the resultant products to be delivered via a low volume subcutaneous injection.

But a combination of the high therapeutic dose and the highly desirable low injection volume often leads to a need for very highly concentrated formulations of the active ingredient. It is well known that achieving stable aqueous formulations of biotherapeutics at high concentration can be exceptionally challenging, often leading to a considerable increase in the rate of aggregation, particle formation and in viscosity. High viscosity is unacceptable as it significantly limits the injectability of the product.

Antibodies and other protein therapeutics may be administered parenterally, such as by intravenous (IV), intramuscular (IM) or subcutaneous (SC) route. Subcutaneous injection has gained increasing attention for the delivery of protein therapeutics due to its potential to simplify patient administration (fast, low-volume injection) and reduced treatment costs (shorter medical assistance). To ensure patient compliance, it is desirable that subcutaneous injection dosage forms be isotonic and include small injection volumes (<2.0 ml per injection site). To reduce injection volume, proteins are often administered within the range of 1 mg/ml to 150 mg/ml.

Thus, primarily development of protein formulations for subcutaneous administration is often associated with viscosity challenges. Volume limitations (< 2 ml) and dose requirements (usually > 100 mg administration) often demand for highly concentrated protein formulations. But at high concentrations, as already said, proteins tend to form highly viscous solutions and the stability can become problematic due to the formation of soluble and insoluble protein-protein aggregates. As such viscosity is a severe challenge for
a) the manufacturing process and
b) the administration to the patient.

In the manufacturing process, highly concentrated protein formulations that are highly viscous, present difficulties in processing, particularly in ultrafiltration and sterile filtration. Furthermore, the increased viscosity yields to increased shear stress to the protein, which frequently yields to loss of product.

mAb-based therapies are usually administered repeatedly over an extended period of time and require several mg/kg dosing. Antibody solutions or suspensions can be administered via parenteral routes, such as by intravenous (IV) infusions, and subcutaneous (SC) or intramuscular (IM) injections. Here, in injection solutions, the high viscosity is a problem. To solve this problem and to improve the stability of the solution, additives and excipients in higher concentrations are usually added as well. At protein concentrations that are desirable for formulations intended for intramuscular or subcutaneous administration, high concentrations of stabilizers, such as sucrose and sodium chloride, are required to achieve long-term protein stability. The resulting solutions often cause injection pain due to high injection forces and to tissue damages.

Therefore, it is critical to balance the needed amounts of stabilizers for stability and osmolarity of the high protein concentration formulations.

As a consequence, the technical hurdles attributed to viscosity oftentimes lead to failure to develop protein formulations for subcutaneous delivery.

In order to increase success rates in the development of subcutaneous formulations, the reduction of and control of viscosity by chemical ways has gained considerable attention in recent years.

A large number of publications and patent applications refer to excipients from the family of salts (mostly NaCl) and of special amino acids, preferably arginine, histidine and proline, which have shown to be efficient in lowering the viscosity of certain high-concentration protein therapeutics.

Unfortunately, these well-known approaches for lowering the viscosity are not universally applicable, probably due to the fact that the viscosity of protein formulations is the result of various intermolecular forces. Depending on the protein molecule and its formulation conditions, different interactions may affect the viscosity, such as molecular crowding, or dipole-dipole or dipole-charge interactions or interactions between hydrophobic or charged groups. Consequently, the pharmaceutical industry has a strong need for viscosity-reducing excipients, especially as an alternative option when standard solutions based on NaCl and amino acids mentioned above, fail.

A high number of viscosity lowering additives and excipients has been researched in the past. At present, however still not all therapeutic proteins solutions exhibiting viscosity issues at high concentration can be adequately addressed by the known viscosity-lowering excipients.

WO2015/038818 A2 discloses a method for reducing viscosity in a liquid pharmaceutical formulation by using camphorsulfonic acid and arginine.

During the bioprocess, the solutions have to be pumped through tubing and chromatography columns. At high viscosities, the flow rate through such columns is limited by said viscosity which leads to longer processing times, significant protein losses during chromatography or might lead to complete non-processability of the protein solution. Furthermore, when passing through a connector from the narrow tube into a less narrow column, shear forces may occur. Shear stress is a typical reason for proteins to denature and potentially to aggregate and thereby reducing the yield of the process. Obviously, such shear stress induced aggregation has an adverse effect on process economics. Moreover, the gel bed within the chromatography column may be damaged by the high pressure.

Additionally, some proteins are formulated into high concentration through tangential flow filtration (TFF). When the viscosity of the solution becomes critical, a gel-like layer may be formed near the membrane. Especially the membrane flux is significantly reduced yielding to an increased processing time and therefore to significant higher manufacturing costs. As discussed before, also during TFF shear stress may occur yielding to insoluble protein aggregates and a reduced yield.

Generally, it has been observed that highly viscous solutions develop a certain stickiness making it difficult to recover the complete solution from containers, out of tubing or to remove the entire substance from processing systems. This loss of substance leads to a significantly reduced product yield with the obvious adverse effect on process economics.

### Object of the present invention

Protein formulations (e.g. monoclonal antibodies, fusion proteins etc.) intended for pharmaceutical applications usually require stabilizers against undesired aggregation and to prevent physical or chemical degradation. These problems are worsened at high protein concentrations, which however are often desirable for therapeutic administration of this class of molecules.

At high concentrations, the proteins tend to self-associate, resulting in high viscosity formulations, and, e.g. complicates the administration of these protein solutions by injection, but also complicating manufacturing processes, in which a tangential flow filtration is often used for the buffer exchange and for the increase of protein concentration. By increasing the back pressure and shear stress during filtration and injection, the therapeutic protein is potentially destabilized or the duration of process times is unduly prolonged. Accordingly, there is a high need within the biopharmaceutical industry for formulation additives and excipients, or combinations thereof, with viscosity lowering properties. However, formulating proteins like monoclonal antibodies requires a careful selection of formulation additives and/or excipients to avoid protein denaturation and loss of biological activity.

But still a high number of emerging new antibodies and antibody-formats require the development of suitable, innovative viscosity lowering additives and/or excipients or of specific additive/excipient combinations or of targeted formulation strategies. These additives/excipients have to be pharmaceutically safe because protein formulations are administered parenterally, which includes the intravenous, intramuscular, intraperitoneal, intradermal or subcutaneous route. Accordingly, the additives which can be used in these formulations must be physiologically compatible and must not have any undesired side effects and must under no circumstances lead to allergic reactions; in particular, they must not cause any anaphylactoid side effects.

Furthermore, a problem to be solved is the provision of excipient combinations that can effectively reduce the viscosity of a protein solution.

Yet another problem to be solved is that many viscosity reducing excipients used at relevant concentrations can adversely affect protein stability. Hence a further problem to be solved is the provision of excipient combinations that can effectively reduce the viscosity of a protein solution and that show an improved protein stability compared to one viscosity reducing excipient alone used in a higher concentration that results in a similar viscosity reduction compared to the combination.

High viscosity of protein solutions causes numerous difficulties in bioprocessing. Since known additives which hitherto are used for reducing the viscosity in corresponding protein solutions do not lead to sufficient viscosity-reducing effects in many cases, it is an object of the present invention to find new possibilities whereby corresponding viscosity-lowering effects can be improved and adverse effects on process economics can be reduced.

### Subject-matter of the invention

Unexpectedly, in experiments for formulating highly concentrated protein formulations excipients are found which solely or in combination with others are suitable for substantially lowering the viscosity.

The present invention refers to a method for reducing the viscosity of a liquid composition comprising a protein in a concentration in the range of at least 50 mg/ml up to 300 mg/ml, comprising the step of combining the liquid composition with with camphorsulfonic acid and at least one cationic excipient, wherein the at least one cationic excipient is selected from the list comprising meglumine, ornithine and carnitine in a concentration with a viscosity-reducing effect.

According to this method a combination of excipients selected from the group of molecules listed in the claims is added to a highly concentrated protein liquid formulation in an optimized concentration. The viscosity of the liquid composition comprising a protein is thereby substantially reduced. A good reduction of the viscosity is achieved when camphorsulfonic acid is added to a liquid composition comprising a protein in combination with at least a cationic excipient selected from the group meglumine, ornithine, and carnitine

Surprisingly, excipient combinations of the present invention can synergistically reduce the viscosity of a liquid protein composition and optionally simultaneously increase stability of the protein.

Surprisingly, in relation to the viscosity reducing potential, protein stability is less negatively influenced when using excipient combinations of the present invention compared to when only one viscosity reducing excipient is used.

The protein can be a therapeutic or pharmaceutically active protein and may be a protein selected from the group of antibodies, antibody fragments, minibody, a nanobody, a modified antibody, antibody-like molecule, antibody-drug conjugate and fusion protein. With this the viscosity of the formulation is reduced by at least 12%, preferably by at least 50%.

Accordingly, an object of the present invention is a liquid protein composition or liquid pharmaceutical formulation obtainable by this method having a reduced viscosity compared to an identical formulation without the excipient combinations.

Good viscosity reductions are achieved with a liquid pharmaceutical formulation comprising a protein, preferably a therapeutic protein in a concentration of at least 40 mg/ml up to 250 mg/ml, preferably in a concentration of at least 90 mg/ml up to 250 mg/ml, and if at least camphorsulfonic acid is added as a viscosity reducing excipient. Especially good viscosity reducing effects are achieved when the concentration of camphorsulfonic acid is less than about 500 mM, especially less than 200 mM in the liquid pharmaceutical composition, which shows a pH in the range between about 3 to about 8, preferably 4.5 to about 8.0, more preferably in the range between about 4.7 to about 7.5, especially preferred in the range of about 5 to about 7.2 and comprising a buffer. Said formulations may comprise a phosphate or acetate buffer. Further, the formulation may comprise a stabilizer. This stabilizer may be a sugar or a surfactant, like sucrose, a polysorbate, preferably polysorbate 80 or poloxamers.

Viscosity reduced pharmaceutical formulations as prepared accordingly may be prepared as a lyophilized powder. A lyophilized powder like this is comprising a therapeutic protein and camphorsulfonic acid, wherein camphorsulfonic acid or a combination of camphorsulfonic acid with a cationic excipient is present in an amount sufficient to upon reconstitution yield to a concentration of less than 500 mM, preferably less than 200 mM and wherein the protein yields to a concentration of at least 40 mg/ml up to 250 mg/ml, preferably at least 90 mg/ml up to 250 mg/ml. Reconstitution of this powder comprises the step of adding a sterile aqueous diluent. Thus, an object of the present invention is a method as characterized by the claims wherein formulations are prepared wherein the therapeutic protein is selected from the group of antibodies, antibody fragments, minibody, a nanobody, a modified antibody, antibody-like molecule and fusion protein, preferably the therapeutic protein is selected from the group of antibodies, antibody fragments, minibody, a nanobody, a modified antibody, antibody-like molecule and fusion protein, and especially preferred the therapeutic protein is selected from the group of antibodies, antibody fragments, minibody, a nanobody, a modified antibody, antibody-like molecule and fusion protein.

An additional disclosure relates to a method for reducing the viscosity of liquid protein compositions in a bioprocess, comprising the step of combining the liquid protein composition with at least camphorsulfonic acid as an excipient in a concentration with a viscosity-reducing effect in the liquid protein composition. Preferably the liquid protein composition is combined with camphorsulfonic acid and at least a cationic excipient. A good reduction of the viscosity is achieved when camphorsulfonic acid is added to liquid protein compositions in combination with at least a cationic excipient selected from the group arginine, meglumine, ornithine, and carnitine

The subject of the present disclosure (not claimed) is also a kit comprising a pharmaceutical formulation as characterized before or comprising a lyophilized powder of in the given embodiments. This kit may comprise freeze-dried or spray-dried preparations of a pharmaceutical composition, obtained by a method as mentioned and which can be made into solution preparations prior to use. Thus, a corresponding kit may comprise ready-to-use freeze-dried or spray-dried formulations sitting in a 96-well plate. The kit may also include containers, syringes and/or other administration devices with or without needles, infusion pumps, jet injectors, pen devices, transdermal injectors, or other needle-free injector and instructions depending on the needs of the application of said kit.

### Detailed description of the invention

As outlined above, high protein concentrations pose challenges relating to the physical and chemical stability of the protein in liquid formulations, as well as difficulties with manufacture, storage, and administration of said protein formulation. A major problem is the tendency of proteins to aggregate and form particulates during processing and/or storage, which makes manipulations during further processing and/or administration difficult. Concentration-dependent degradation and/or aggregation are major challenges in developing liquid protein formulations at higher concentrations. In addition to the potential for non-native protein aggregation and particle formation, reversible self-association in aqueous solutions may occur, which contributes to, among other things, increased viscosity that complicates delivery by injection.

### Definitions

The term "protein," as generally used herein, refers to a polymer of amino acids linked to each other by peptide bonds to form a polypeptide for which the chain length is sufficient to produce at least a detectable tertiary structure. Proteins having a molecular weight (expressed in kDa wherein "Da" stands for "Daltons" and 1 kDa=1,000 Da) greater than about 100 kDa may be designated "high-molecular-weight proteins," whereas proteins having a molecular weight less than about 100 kDa may be designated as "low-molecular-weight proteins." The term "low-molecular-weight protein" excludes small peptides lacking the requisite of at least tertiary structure necessary to be considered a protein. Protein molecular weight may be determined using standard methods known to one skilled in the art, including, but not limited to, mass spectrometry (e.g., ESI, MALDI) or calculation from known amino acid sequences and glycosylation. Proteins can be naturally occurring or non-naturally occurring, synthetic, or semi-synthetic.

"Essentially pure protein(s)" and "substantially pure protein(s)" are used interchangeably herein and refer to a composition comprising at least about 90% by weight pure protein, preferably at least about 95% pure protein by weight. "Essentially homogeneous" and "substantially homogeneous" are used interchangeably herein and refer to a composition wherein at least about 90% by weight of the protein present is a combination of the monomer and reversible di- and oligomeric associates (not irreversible aggregates), preferably at least about 95%.

The term "antibody," as generally used herein, broadly covers mAbs (including full-length antibodies which have an immunoglobulin Fc region), antibody compositions with polyepitopic specificity, bispecific antibodies, diabodies, and single-chain antibody molecules, as well as antibody fragments (e.g., Fab, Fab', F(ab')2, and Fv), single domain antibodies, multivalent single domain antibodies, Fab fusion proteins, and fusions thereof.

The term "monoclonal antibody" or "mAb," as generally used herein, refers to an antibody obtained from a population of substantially homogeneous antibodies, i.e., the individual antibodies comprising the population are identical, except for possible naturally occurring mutations that may be present in minor amounts. Monoclonal antibodies are highly specific, being directed against a single epitope. These are typically synthesized by culturing hybridoma cells, as described by Kohler et al. (Nature 256: 495, 1975), or may be made by recombinant DNA methods (see, e.g., U.S. Pat. No. 4,816,567), or isolated from phage antibody libraries using the techniques described in Clackson et al. (Nature 352: 624-628, 1991) and Marks et al. (J. Mol. Biol. 222: 581-597, 1991), for example. As used herein, "mAbs" specifically include derivatized antibodies, antibody-drug conjugates, and "chimeric" antibodies in which a portion of the heavy and/or light chain is identical with or homologous to corresponding sequences in antibodies derived from a particular species or belonging to a particular antibody class or subclass, while the remainder of the chain(s) is (are) identical with or homologous to corresponding sequences in antibodies derived from another species or belonging to another antibody class or subclass, as well as fragments of such antibodies, so long as they exhibit the desired biological activity (U.S. Pat. No. 4,816,567; Morrison et al., Proc. Natl. Acad. Sci. USA 81:6851-6855, 1984).

An "antibody fragment" comprises a portion of an intact antibody, including the antigen binding and/or the variable region of the intact antibody. Examples of antibody fragments include Fab, Fab', F(ab')2, and Fv fragments; diabodies; linear antibodies (see U.S. Pat. No. 5,641,870; Zapata et al., Protein Eng. 8:1057-1062, 1995); single-chain antibody molecules; multivalent single domain antibodies; and multi-specific antibodies formed from antibody fragments.

"Humanized" forms of non-human (e.g., murine) antibodies are chimeric immunoglobulins, immunoglobulin chains, or fragments thereof (such as Fv, Fab, Fab', F(ab')2, or other antigen-binding subsequences of antibodies) of mostly human sequences, which contain minimal sequences derived from non-human immunoglobulin. (See, e.g., Jones et al., Nature 321:522-525, 1986; Reichmann et al., Nature 332:323-329, 1988; and Presta, Curr. Op. Struct. Biol. 2:593-596, 1992.)

In this context, the term "therapeutically active protein", "pharmaceutically active protein" or "therapeutic protein" refers to a protein or peptide as defined above that is administered to a subject with the aim of treating or preventing a disease or medical condition. In particular, the subject may be a mammal or a human. Therapeutic proteins can be administered for different purposes, such as replacing a protein that is deficient or abnormal, augmenting an existing pathway, providing a novel function or activity, interfering with a molecule or organism and delivering other compounds or proteins, such as a radionuclide, cytotoxic drug, or effector proteins. Therapeutic proteins encompass antibody-based drugs, Fc fusion proteins, anticoagulants, blood factors, bone morphogenetic proteins, engineered protein scaffolds, enzymes, growth factors, hormones, interferons, interleukins, antibody drug conjugates (ADCs) and thrombolytics. Therapeutic proteins can be naturally occurring proteins or recombinant proteins. Their sequence can be natural or engineered.

"Rheology" refers to the study of the deformation and flow of matter and "viscosity" refers to the resistance of a substance (typically a liquid) to flow. Viscosity is related to the concept of shear force; it can be understood as the effect of different layers of the fluid exerting shearing force on each other, or on other surfaces, as they move against each other. There are several measures of viscosity. The units of viscosity are Ns/m², known as Pascal-seconds (Pa-s). Viscosity can be "kinematic" or "absolute". Kinematic viscosity is a measure of the rate at which momentum is transferred through a fluid. It is measured in Stokes (St). The kinematic viscosity is a measure of the resistive flow of a fluid under the influence of gravity. When two fluids of equal volume and differing viscosity are placed in identical capillary viscometers and allowed to flow by gravity, the more viscous fluid takes longer than the less viscous fluid to flow through the capillary. If, for example, one fluid takes 200 seconds (s) to complete its flow and another fluid takes 400 s, the second fluid is called twice as viscous as the first on a kinematic viscosity scale. The dimension of kinematic viscosity is length²/time. Commonly, kinematic viscosity is expressed in centiStokes (cSt). The SI unit of kinematic viscosity is mm²/s, which is equal to 1 cSt. The "absolute viscosity," sometimes called "dynamic viscosity" or "simple viscosity," is the product of kinematic viscosity and fluid density. Absolute viscosity is expressed in units of centipoise (cP). The SI unit of absolute viscosity is the milliPascal-second (mPa-s), where 1 cP=1 mPa-s.

Viscosity may be measured by using, for example, a viscometer at a given shear rate or multiple shear rates. An "extrapolated zero-shear" viscosity can be determined by creating a best fit line of the four highest-shear points on a plot of absolute viscosity versus shear rate, and linearly extrapolating viscosity back to zero-shear. Alternatively, for a Newtonian fluid, viscosity can be determined by averaging viscosity values at multiple shear rates. Viscosity can also be measured using a microfluidic viscometer at single or multiple shear rates (also called flow rates), wherein absolute viscosity is derived from a change in pressure as a liquid flows through a channel. Viscosity equals shear stress over shear rate. Viscosities measured with microfluidic viscometers can, in some embodiments, be directly compared to extrapolated zero-shear viscosities, for example those extrapolated from viscosities measured at multiple shear rates using a cone and plate viscometer. According to the invention, viscosity of compositions and formulations is reduced when at least one of the methods described above show a viscosity lowering effect. Preferably, viscosity is measured using mVROC^{™} Technology. More preferably the viscosity is measured using mVROC^{™} Technology at 20 °C. Most preferably the viscosity is measured at 20 °C using mVROC^{™} Technology and using a 500 µl syringe, a shear rate of 3000 s-1 or 2000 s-1 and a volume of 200 µl. The person ordinary skilled in the art is familiar with the viscosity measurement using mVROC^{™} Technology, especially with selecting the parameters described above. Detailed specifications, methods and setting can be found in the 901003.5.1-mVROC_User's_Manual.

"Shear rate" refers to the rate of change of velocity at which one layer of fluid passes over an adjacent layer. The velocity gradient is the rate of change of velocity with distance from the plates. This simple case shows the uniform velocity gradient with shear rate (v₁-v₂)/h in units of (cm/sec)/(cm)=1/sec. Hence, shear rate units are reciprocal seconds or, in general, reciprocal time. For a microfluidic viscometer, change in pressure and flow rate are related to shear rate. "Shear rate" is to the speed with which a material is deformed. Formulations containing proteins and viscosity-lowering agents are typically measured at shear rates ranging from about 0.5 s⁻¹ to about 200 s⁻¹ when measured using a cone and plate viscometer and a spindle appropriately chosen by one skilled in the art to accurately measure viscosities in the viscosity range of the sample of interest (i.e., a sample of 20 cP is most accurately measured on a CPE40 spindle affixed to a DV2T viscometer (Brookfield)); greater than about 20 s⁻¹ to about 3,000 s⁻¹ when measured using a microfluidic viscometer.

For classical "Newtonian" fluids, as generally used herein, viscosity is essentially independent of shear rate. For "non-Newtonian fluids," however, viscosity either decreases or increases with increasing shear rate, e.g., the fluids are "shear thinning" or "shear thickening", respectively. In the case of concentrated (i.e., high-concentration) protein solutions, this may manifest as pseudoplastic shear-thinning behavior, i.e., a decrease in viscosity with shear rate.

The term "chemical stability," as generally used herein, refers to the ability of the protein components in a formulation to resist degradation via chemical pathways, such as oxidation, deamidation, or hydrolysis. A protein formulation is typically considered chemically stable if less than about 5% of the components are degraded after 24 months at 4° C.

The term "physical stability," as generally used herein, refers to the ability of a protein formulation to resist physical deterioration, such as aggregation. A formulation that is physically stable forms only an acceptable percentage of irreversible aggregates (e.g., dimers, trimers, or other aggregates) of the bioactive protein agent. The presence of aggregates may be assessed in several ways, including by measuring the average particle size of the proteins in the formulation by means of dynamic light scattering. A formulation is considered physically stable if less than about 5% irreversible aggregates are formed after 24 months at 4° C. Acceptable levels of aggregated contaminants ideally would be less than about 2%. Levels as low as about 0.2% are achievable, although approximately 1% is more typical.

The term "stable formulation," as generally used herein, means that a formulation is both chemically stable and physically stable. A stable formulation may be one in which more than about 95% of the bioactive protein molecules retain bioactivity in a formulation after 24 months of storage at 4° C., or equivalent solution conditions at an elevated temperature, such as one month storage at 40° C. Various analytical techniques for measuring protein stability are available in the art and are reviewed, for example, in Peptide and Protein Drug Delivery, 247-301, Vincent Lee, Ed., Marcel Dekker, Inc., New York, N.Y. (1991) and Jones, A., Adv. Drug Delivery Revs. 10:29-90, 1993. Stability can be measured at a selected temperature for a certain time period. For rapid screening, for example, the formulation may be kept at 40° C., for 2 weeks to one month, at which time residual biological activity is measured and compared to the initial condition to assess stability. When the formulation is to be stored at
2 - 8 °C., generally the formulation should be stable at 30° C. or 40° C. for at least one month and/or stable at 2° C.-8° C. for at least 2 years. When the formulation is to be stored at room temperature, about 25° C., generally the formulation should be stable for at least 2 years at about 25° C. and/or stable at 40° C. for at least about 6 months. The extent of aggregation following lyophilization and storage can be used as an indicator of protein stability. In some instances, the stability is assessed by measuring the particle size of the proteins in the formulation. In some instances, stability may be assessed by measuring the activity of a formulation using standard biological activity or binding assays well within the abilities of one ordinarily skilled in the art.

The term protein "particle size," as generally used herein, means the average diameter of the predominant population of bioactive molecule particulates, or particle size distributions thereof, in a formulation as determined by using well known particle sizing instruments, for example, dynamic light scattering, SEC (size exclusion chromatography), or other methods known to one ordinarily skilled in the art.

The term "concentrated" or "high-concentration", as generally used herein, describes liquid protein formulations having a final concentration of protein of at least 1 mg/ml, especially greater than about 10 mg/mL, preferably greater than about 50 mg/mL, more preferably greater than about 100 mg/mL, still more preferably greater than about 200 mg/mL, or most preferably greater than about 250 mg/mL.

A "reconstituted formulation," as generally used herein, refers to a formulation which has been prepared by dissolving a dry powder, lyophilized, spray-dried or solvent-precipitated protein in a diluent, such that the protein is dissolved or dispersed in aqueous solution for administration.

A "lyoprotectant" is a substance which, when combined with a protein, significantly reduces chemical and/or physical instability of the protein upon lyophilization and/or subsequent storage. The lyoprotectant is generally added to the pre-lyophilized formulation in a "lyoprotecting amount." This means that, following lyophilization of the protein in the presence of the lyoprotecting amount of the lyoprotectant, the protein essentially retains its physical and chemical stability and integrity.

A "diluent" or "carrier," as generally used herein, is a pharmaceutically acceptable (i.e., safe and non-toxic for administration to a human or another mammal) and useful ingredient for the preparation of a liquid formulation, such as an aqueous formulation reconstituted after lyophilization. Exemplary diluents include sterile water, bacteriostatic water for injection (BWFI), a pH buffered solution (e.g., phosphate-buffered saline), sterile saline solution, Ringer's solution or dextrose solution, and combinations thereof.

A "preservative" is a compound which can be added to the formulations herein to reduce contamination by and/or action of bacteria, fungi, or another infectious agent. The addition of a preservative may, for example, facilitate the production of a multi-use (multiple-dose) formulation.

A "bulking agent," as generally used herein, is a compound which adds mass to a lyophilized mixture and contributes to the physical structure of the lyophilized cake (e.g. facilitates the production of an essentially uniform lyophilized cake which maintains an open pore structure).

A "therapeutically effective amount" is the least concentration required to effect a measurable improvement or prevention of any symptom or a particular condition or disorder, to effect a measurable enhancement of life expectancy, or to generally improve patient quality of life. The therapeutically effective amount is dependent upon the specific biologically active molecule and the specific condition or disorder to be treated. Therapeutically effective amounts of many proteins, such as the mAbs described herein, are well known in the art. The therapeutically effective amounts of proteins not yet established or for treating specific disorders with known proteins, such as mAbs, to be clinically applied to treat additional disorders may be determined by standard techniques which are well within the craft of a skilled artisan, such as a physician.

The term "injectability" or "syringeability," as generally used herein, refers to the injection performance of a pharmaceutical formulation through a syringe equipped with an 18-32-gauge needle, optionally thin walled. Injectability depends upon factors such as pressure or force required for injection, evenness of flow, aspiration qualities, and freedom from clogging. Injectability of the liquid pharmaceutical formulations may be assessed by comparing the injection force of a reduced-viscosity formulation to a standard formulation without added viscosity-lowering agents. The reduction in the injection force of the formulation containing a viscosity-lowering agent reflects improved injectability of that formulation. The reduced viscosity formulations have improved injectability when the injection force is reduced by at least 10%, preferably by at least 30%, more preferably by at least 50%, and most preferably by at least 75% when compared to a standard formulation having the same concentration of protein under otherwise the same conditions, except for replacement of the viscosity-lowering agent with an appropriate buffer of about the same concentration. Alternatively, injectability of the liquid pharmaceutical formulations may be assessed by comparing the time required to inject the same volume, such as 0.5 mL, or more preferably about 1 mL, of different liquid protein formulations when the syringe is depressed with the same force.

The term "injection force," as generally used herein, refers to the force required to push a given liquid formulation through a given syringe equipped with a given needle gauge at a given injection speed. The injection force is typically reported in Newtons. For example, the injection force may be measured as the force required to push a liquid formulation through a 1 mL plastic syringe having a 0.25 inch inside diameter, equipped with a 0.50-inch 27-gauge needle at a 250 mm/min injection speed. Testing equipment can be used to measure the injection force. When measured under the same conditions, a formulation with lower viscosity will generally require an overall lower injection force.

The term "reduced-viscosity formulation," as generally used herein, refers to a liquid formulation having a high concentration of a high-molecular-weight protein, such as a mAb, or a low-molecular-weight protein that is modified by the presence of one or more additives to lower the viscosity, as compared to a corresponding formulation that does not contain the viscosity-lowering additive(s) or agent(s).

The term "viscosity-lowering agent," as used herein, refers to a compound which acts to reduce the viscosity of a solution relative to the viscosity of the solution absent the viscosity-lowering agent. The viscosity-lowering agent may be a single compound or may be a mixture of one or more compounds. When the viscosity-lowering agent is a mixture of two or more compounds, the listed concentration refers to each individual agent, unless otherwise specified. By way of example, a formulation containing about 0.25 M meglumine benzenesulfonate as the viscosity-lowering agent is a solution having benzenesulfonic acid at a concentration of 0.25 M, and meglumine at a concentration of 0.25 M.

Certain viscosity-lowering agents contain acidic or basic functional groups and may show hydrophilic and hydrophobic regions, which together influence the interaction characteristics with comprising proteins of the solution. Whether or not the functional groups are fully or partially ionized depends on the pH of the formulation they are in. Unless otherwise specified, reference to a formulation containing a viscosity-lowering agent having an ionizable functional group includes both the parent compound and any possible ionized states.

The term "liquid formulation" or "formulation" as used herein, is a protein that is either supplied in an acceptable pharmaceutical diluent or one that is reconstituted in an acceptable pharmaceutical diluent prior to administration to the patient.

Biosimilars can be produced by microbial cells (prokaryotic, eukaryotic), cell lines of human or animal origin (e.g., mammalian, avian, insect), or tissues derived from animals or plants. The expression construct for a proposed biosimilar product will generally encode the same primary amino acid sequence as its reference product. Minor modifications, such as N- or C-terminal truncations that will not have an effect on safety, purity, or potency, may be present.

A biosimilar mAb is similar to the reference mAb physiochemically or biologically both in terms of safety and efficacy. The biosimilar mAb can be evaluated against a reference mAb using one or more in vitro studies including assays detailing binding to target antigen(s); binding to isoforms of the Fc gamma receptors (FcyRI, FcyRII, and FcγRIII), FcRn, and complement (C1q); Fab-associated functions (e.g. neutralization of a soluble ligand, receptor activation or blockade); or Fc-associated functions (e.g. antibody-dependent cell-mediated cytotoxicity, complement-dependent cytotoxicity, complement activation). In vitro comparisons may be combined with in vivo data demonstrating similarity of pharmacokinetics, pharmacodynamics, and/or safety. Clinical evaluations of a biosimilar mAb against a reference mAb can include comparisons of pharmacokinetic properties (e.g. AUC_{0-inf}, AUC₀₋ₜ, Cₘₐₓ, tₘₐₓ, C_{trough}); pharmacodynamic endpoints; or similarity of clinical efficacy (e.g. using randomized, parallel group comparative clinical trials). The quality comparison between a biosimilar mAb and a reference mAb can be evaluated using established procedures, including those described in the "Guideline on similar biological medicinal products containing biotechnology-derived proteins as active substance: Quality issues" (EMEA/CHMP/BWP/49348/2005), and the "Guideline on development, production, characterization and specifications for monoclonal antibodies and related substances" (EMEA/CHMP/BWP/157653/2007).

Differences between a biosimilar mAb and a reference mAb can include post-translational modification, e.g. by attaching to the mAb other biochemical groups such as a phosphate, various lipids and carbohydrates; by proteolytic cleavage following translation; by changing the chemical nature of an amino acid (e.g., formylation); or by many other mechanisms. Other post-translational modifications can be a consequence of manufacturing process operations - for example, glycation may occur with exposure of the product to reducing sugars. In other cases, storage conditions may be permissive for certain degradation pathways such as oxidation, deamidation, or aggregation, as all these product-related variants may be included in a biosimilar mAb.

As used herein, the term "pharmaceutically acceptable salts" refers to salts prepared from pharmaceutically acceptable non-toxic acids and bases, including inorganic acids and bases, and organic acids and bases.

As used herein, term "alkyl group" refers to straight-chain, branched-chain and cyclic hydrocarbon groups. Unless specified otherwise, the term alkyl group embraces hydrocarbon groups containing one or more double or triple bonds. An alkyl group containing at least one ring system is a "cycloalkyl" group. An alkyl group containing at least one double bond is an "alkenyl group," and an alkyl group containing at least one triple bond is an "alkynyl group."

The term as used herein, "Aryl" refers to aromatic carbon ring systems, including fused ring systems. In an "aryl" group, each of the atoms that form the ring are carbon atoms.

As used herein "Heteroaryl" refers to aromatic ring systems, including fused ring systems, wherein at least one of the atoms that forms the ring is a heteroatom. Furthermore, the term as used herein "Heterocycle" refers to ring systems that, including fused ring systems, are not aromatic, wherein at least one of the atoms that forms the ring is a heteroatom.

The term as used herein, "heteroatom" is any non-carbon or non-hydrogen atom. Preferred heteroatoms include oxygen, sulfur, and nitrogen.

The term "bioprocess" refers to therapeutic cell manufacturing processes, which can be separated into upstream processes and downstream processes. The upstream process is defined as the entire process prior to separating protein from cellular compounds. The upstream process comprises early cell isolation and cultivation, to cell banking and culture expansion of the cells until final harvest. The downstream part of a bioprocess refers to the part where the target protein is purified from the feed of the upstream and is processed to meet purity and quality requirements. Some type of cells need to be disrupted when entering the downstream process. Yet other cells may secrete the target protein into the media and need to be removed via filtration. Further downstream processing is usually divided into the main sections: a purification section and a polishing section. A bioprocess can be a batch process or a semi-continuous or a continuous process.

The term "permeate flux" refers to the volume passing through a defined filter within a certain period of time, typically on the order of minutes.

The term "filtration step" refers to a process step where a liquid is passed through a material with a defined pore size allowing for the separation of materials based on their size. For some filters the pore size is defined in nanometers. Yet for other filters, the pore size is not directly defined, but the weight of a molecule to be withheld is given. Filtering materials can be placed in a way that they block the cross-section of the filtration device (dead-end filtration). Yet filtering materials can be placed in a way that the solution to be filtered is tangentially flowing across the surface of said material, e.g. tangential flow filtration. The filtering material can be a membrane, a glass filter, a metallic filter or a resin. The resin can be held in a chromatography column. The resin can be a cationic or anion exchange resin, an affinity resin, like a Protein A or glutathione resin, or a hydrophobic or hydrophilic resin.

The term "protein recovery" after buffer exchange and volume reduction refers to the fraction of protein to be retrieved after a process step.

The term "tangential flow filtration" or "TFF" refers to a method of filtration where a solution passes over a defined filter tangentially. Substances smaller than the filter pores are forced out of the solution through the filter by the pressure resulting from solution flow rate, viscosity, temperature and other factors.

### Formulations

Biocompatible, low-viscosity protein solutions, such as those of mAbs, can be used to deliver therapeutically effective amounts of proteins in volumes useful for subcutaneous (SC) and intramuscular (IM) injections, typically less than or about 2 ml for SC and less than or about 5 ml for IM, more preferably less than or about 1 ml for SC and less than or about 3 ml for IM. The proteins can generally have any molecular weight, although in some instances, high-molecular-weight proteins are preferred. In other instances, the proteins are low-molecular-weight proteins.

Now, the present invention provides a method of reducing the viscosity and optionally improving stability of a liquid composition comprising a protein, comprising the step of combining the liquid composition with a viscosity-reducing amount of camphorsulfonic acid as an excipient combined with at least a suitable a cationic excipient selected from the group meglumine, ornithine and carnitine.

These excipients are added in suitable amounts to the formulations. Preferably they are added in equimolar amounts to the comprising liquid protein compositions. Depending on the pH value of the solution, the concentration of the liquid protein composition, the nature of the protein, the resulting concentration of the added excipient(s) and its (their) chemical nature the viscosity reducing effect varies. In a particular embodiment the liquid composition is a liquid pharmaceutical formulation and the protein is a therapeutic protein.

In particular, when camphorsulfonic acid and at least one cationic excipients are added in equimolar amounts to the concentrated liquid protein composition, for example to solutions of (mAbC, mAbD and mAbE), a particularly good viscosity reduction is achieved. According to the present invention mAbC represents the monoclonal antibody Infliximab, mAbD represents the monoclonal antibody Evolocumab and mAbE represents the monoclonal antibody Reslizumab. Unexpectedly, it was found by experiments that mixtures of camphorsulphonic acid in combination with cationic acids selected from the group arginine, meglumine, ornithine, and carnitine as specific equimolar mixtures significantly reduce the viscosity of highly concentrated protein liquid formulations of monoclonal antibodies or of fusion proteins.

Furthermore, it was found that mixtures of camphorsulphonic acid in combination with cationic acids selected from the group arginine, meglumine, ornithine, and carnitine can synergically reduce the viscosity and/or increase stability in the compositions and formulations comprising a protein. As defined herein, "synergically" refers to the effect that the action of a combination of components is greater than the sum of the action of each of the components alone.

The present invention provides a method for synergistically reducing the viscosity of a liquid composition comprising a protein or a liquid formulation comprising a pharmaceutically active protein in a concentration in the range of at least 50 mg/ml up to 300 mg/ml, comprising the step of combining the liquid protein composition with at least camphorsulfonic acid as an excipient in combination with cationic acids selected from the group meglumine, ornithine, and carnitine in a concentration with a viscosity-reducing effect. In a particular disclosure (not claimed by the present invention) the viscosity is synergistically reduced by a combination of camphorsulfonic acid and arginine. Preferably the viscosity is synergistically reduced by a combination of camphorsulfonic acid and arginine in a concentration ratio of 1:1.

Furthermore, it was surprisingly found that the addition of cationic acids selected from the group arginine, meglumine, ornithine, and carnitine to camphorsulphonic acid results in an improved stability of the protein compared to the camphorsulfonic acid alone.

The disclosure further provides a method for stabilizing a protein in a liquid composition or a pharmaceutically active protein in a liquid formulation in a concentration in the range of at least 50 mg/ml up to 300 mg/ml, comprising the step of combining the liquid protein composition with at least camphorsulfonic acid as an excipient in combination with cationic acids selected from the group arginine, meglumine, ornithine, and carnitine in a concentration with a viscosity-reducing effect. In a particular embodiment the protein is stabilized by a combination of camphorsulfonic acid and arginine or camphorsulfonic acid and ornithine. Preferably the protein is stabilized by a combination of camphorsulfonic acid and arginine or camphorsulfonic acid and ornithine in a concentration ratio of 1:1. Preferably the stability of the pharmaceutically active protein is improved by at least 5%, 10%, 15%, 20%, 25%, 30%, 35%, 40%, 45%, 50%, 55%, 60%, 65%, 70% or 75% compared to a liquid composition comprising a protein or a liquid formulation comprising a pharmaceutically active protein in a concentration in the range of at least 50 mg/ml up to 300 mg/ml, comprising the step of combining the liquid protein composition with camphorsulfonic acid as an excipient.

The disclosure (not part of the present invention) further provides a method for synergistically reducing the viscosity of a liquid composition comprising a protein or a liquid formulation comprising a pharmaceutically active protein in a concentration in the range of at least 50 mg/ml up to 300 mg/ml, comprising the step of combining the liquid protein composition with camphorsulfonic acid as an excipient in combination with arginine in a concentration with a viscosity-reducing effect, whereas the stability of the pharmaceutically active protein is improved by at least 5%, 10%, 15%, 20%, 25%, 30%, 35%, 40%, 45%, 50%, 55%, 60%, 65%, 70% or 75% compared to a liquid composition comprising a protein or a liquid formulation comprising a pharmaceutically active protein in a concentration in the range of at least 50 mg/ml up to 300 mg/ml, comprising the step of combining the liquid protein composition with camphorsulfonic acid as an excipient.

In exemplary embodiments, the protein or therapeutic protein is at a high protein concentration as described above. In some embodiments, the reduction in viscosity is at least about 5%, 10%, 15%, 20%, 25%, 30%, 35%, 40%, 45%, 50%, 55%, 60%, 65% or 70% compared to control formulations in which buffer solution was added to the liquid protein composition in the same amount instead of the viscosity reducing agent solution.

In exemplary embodiments, the protein or therapeutic protein is at a high protein concentration as described above of at least 50 mg/ml, preferably more than 75 mg/ml, more preferable more than 100 mg/ml. Formulations tested and disclosed here have protein concentrations in the range of 150 -280 mg/ml. In some embodiments, the reduction in viscosity is at least about 5%, 10%, 15%, 20%, 25%, 30%, 35%, 40%, 45%, 50%, 55%, 60%, 65%, 70% or 75% compared to control formulations or more.

In another aspect, the disclosure provides liquid solutions comprising a therapeutic protein and camphorsulfonic acid as an excipient and at least further cationic excipients selected from the group consisting of arginine, meglumine, ornithine, and carnitine or mixtures thereof wherein the formulations exhibit reduced viscosity relative to control formulations. In exemplary embodiments, the therapeutic protein is at a high protein concentration as described above, and the excipient(s) described herein is present at a viscosity-reducing concentration. Camphorsulfonic acid and its excipient(s) can be used at concentrations up to their solubility limit. Such solutions may further comprise other additives in an amount effective to further improve stability, reduce aggregation, and/or make the formulation isotonic, without significantly increasing viscosity.

In further embodiments, the concentration of camphorsulphonic acid as the excipient is less than about 500 mM, especially less than 200 mM. Preferably the concentration of camphorsulphonic acid is at least about 50 mM to about 300 mM, or at least about 100 mM to about 250 mM, or at least about 140 mM to about 200 mM. In exemplary embodiments the concentration of the excipient is at least about 50, 100, 105, 110, 115, 120, 125, 130, 135, 140, 145, 150, 155, 160, 165, 170, 175, 180, 185, 190, 195, 200, 210, 220, 250, or 300 mM or greater. The concentration of the at least one cationic excipient selected from the group consisting of meglumine, ornithine, and carnitine or mixtures thereof is at least about 50 mM to about 300 mM, or at least about 100 mM to about 250 mM, or at least about 140 mM to about 200 mM. In exemplary embodiments the concentration of the at least one cationic excipient is at least about 50, 100, 105, 110, 115, 120, 125, 130, 135, 140, 145, 150, 155, 160, 165, 170, 175, 180, 185, 190, 195, 200, 210, 220, 250, or 300 mM or greater. Preferred concentration of camphorsulphonic acid is between 25 mM and 250 mM, more preferred between 50 mM and 200 mM, most preferred between 75 mM and 150 mM when used alone or when in combination with at least one cationic excipient. When used in combination the concentration of the at least one cationic excipient is preferably between 25 mM and 250 mM, more preferred between 50 mM and 200 mM, most preferred between 75 mM and 150 mM. When camphorsulfonic acid is used in combination with a cationic excipient selected from the group carnitine. meglumine and ornithine, the molar ratio between camphorsulfonic acid and the cationic excipient is preferably in the range of 1:3 to 3:1, more preferred 1:2 to 2:1, most preferred 1:1. For example, a combination of 25 mM carnitine with 50 mM camphorsulfonic acid yields to an excipient concentration of 75 mM in solution of a 1 : 2 mixture of these excipients. Other exemplary embodiments include concentrations of excipients effective to make the formulation isotonic, without significantly increasing viscosity. Exemplary concentrations include those at about 150 mM or higher, in further embodiments the amounts are at least about 170 mM or higher. However, the concentration chosen has to be so that it results in an effective reduction of the viscosity in the liquid protein composition, and the selected concentration stays safe and tolerable for the organism.

In another aspect, the disclosure provides lyophilized protein formulations comprising a therapeutic protein together with camphorsulfonic acid as an excipient and at least a cationic excipient selected from the group consisting of arginine, meglumine, ornithine, and carnitine or mixtures thereof wherein upon reconstitution with the recommended amount of diluent, the formulations exhibit reduced viscosity relative to control formulations. In exemplary embodiments, the therapeutic protein is at a high protein concentration as described above. In some embodiments, the excipient is present at an amount effective to reduce viscosity upon reconstitution with diluent, for example comprising 98 mg mAbC: 150 mM excipient. Such formulations may comprise further additives, in an amount effective to further improve stability, reduce aggregation, and/or make the formulation isotonic, without significantly increasing viscosity.

In exemplary embodiments of the present invention, the concentration of the excipient(s) and the selected excipient is at least about 1 µg per mg therapeutic protein, up to about 1.0 mg per mg therapeutic protein. In some embodiments, the concentration of excipient is at least about 1, 10, 50, 100, 150, 200, 250, 300, 350, 400, 450, 500 or 550 µg per mg therapeutic protein. In other exemplary embodiments, the concentration of excipient is up to about 600, 650, 700, 750, 800, 850, 900, 950 or 1000 µg per mg therapeutic protein.

The present disclosure further provides a method of preventing self-association of proteins in liquid formulations by using camphorsulfonic acid in combination with at least a cationic excipient selected from the group of arginine, meglumine, ornithine, and carnitine or mixtures thereof as excipient(s) in any of the amounts or concentrations described herein.

The disclosure (not part of the present invention) also provides a kit comprising a liquid protein formulation of the invention, and instructions for its administration, optionally with a container, syringe and/or other administration device. The invention further provides a kit comprising a lyophilized protein formulation of the invention, optionally in a container, and instructions for its reconstitution and administration, optionally with a vial of sterile diluent, and optionally with a syringe or other administration device. Exemplary containers include vials, tubes, bottles, single or multi-chambered prefilled syringes, or cartridges, but also a 96-well plate comprising ready-to-use freeze-dried or spray-dried formulations sitting in the wells. Exemplary administration devices include syringes, with or without needles, infusion pumps, jet injectors, pen devices, transdermal injectors, or other needle-free injectors.

Another aspect of the present disclosure (not part of the present invention) is to provide a method for screening for a viscosity-reducing concentration of an excipient_comprising the steps of: (1) assessing the viscosity of a first solution comprising a first concentration of camphorsulphonic acid as an excipient and of a suitable excipient selected from the group consisting of of arginine, meglumine, ornithine, and carnitine or mixtures thereof and a therapeutic protein, such as an antibody, (2) assessing the viscosity of a second solution comprising a different second concentration of the excipient and the therapeutic protein, and (3) determining that the first concentration of excipient is more viscosity-reducing than the second concentration of excipient if the first solution is less viscous. Viscosity can be determined, e.g., using a m-VROC^{™} Technology rheometer (RheoSense, San Ramon, California, USA) or an Aries ARG2 Rheometer or a Brookfield RV-DVIII Rheometer.

Similar methods are provided for screening for an aggregation-reducing or stabilizing concentration of an excipient.

Stability can be assessed in many ways, including monitoring conformational change over a range of temperatures (thermostability) and/or time periods (shelf-life) and/or after exposure to stressful handling situations (e.g. physical shaking). Stability of formulations containing varying concentrations of formulation components can be measured using a variety of methods. For example, the amount of protein aggregation can be measured by visual observation of turbidity, by measuring absorbance at a specific wavelength, by size exclusion chromatography (in which aggregates of a protein will elute in different fractions compared to the protein in its native active state), HPLC, or other chromatographic methods. Other methods of measuring conformational change can be used, including using differential scanning calorimetry (DSC), e.g. to determine the temperature of denaturation, or circular dichroism (CD), which measures the molar ellipticity of the protein. Fluorescence can also be used to analyze the composition. Fluorescence encompasses the emission of light subsequent to absorption of light, which requires a suitable wavelength. Potential readouts are changes in the polar properties of light, light intensity, or emission wavelength. Fluorescence emission can be intrinsic to a protein or can be due to a fluorescence reporter molecule, that for example binds to the hydrophobic pockets of partially unfolded proteins. An increase in binding of reporter molecules can be monitored by detection of the fluorescence signal of a protein sample. Other means for measuring stability can be used and are well known to persons of skill in the art. According to the disclosure, stability of compositions and formulations is increased when at least one of the methods described above show a stabilizing effect.

In experiments carried out, first, the viscosity lowering potential of camphorsulphonic acid alone is tested and in combination with antibodies (mAbC, mAbD, mAbE. The concentrations of the proteins are adjusted as given in the examples below to create high viscosity levels.

As already described above, the pH value of these formulations is of particular importance for their effectiveness and the usability of the respective pharmaceutically active protein. It is therefore desirable that the pH of the protein formulations investigated is adjusted in the range of between about 3 to about 8, preferable 4.5 to about 8.0. Depending on the nature of the containing protein or peptide the pH value is adjusted preferably in a range between about 3 to about 8, preferable 4.5 to about 5.5 or in a range between about 5.0 to about 8.0. The buffers used to adjust the pH are preferably an acetate buffer (25 mM) at pH 5.0 and phosphate buffered saline (10 mM) at pH 7,2 or 7,5. If necessary, however, another buffer can be used, which is compatible with the contained pharmaceutically active protein and physiologically acceptable.

The concentrations of the viscosity lowering agents are adjusted in a range from between 50 mM towards 500 mM. A chip-based (micro-electro-mechanical system) capillary rheometer, m-VROC^{™} (RheoSence, San Ramon, CA), was employed to measure the dynamic viscosity. In general, the dynamic viscosity which is also referred to as absolute viscosity (coefficient of absolute viscosity) is a measure of internal resistance which can be determined by the self-association of the protein molecules within a highly concentrated solution.

Determined viscosities clearly indicate, that applying camphorsulfonic acid at a certain concentration together with a specific antibody in solution results in a measurable significant reduction of viscosity of highly concentrated liquid protein compositions.

However, particularly unexpectedly the experiments have shown, that the combined addition of camphorsulfonic acid and of a excipient selected from the group of arginine, meglumine, ornithine, and carnitine or mixtures thereof leads to a significantly higher viscosity reduction.

As already pointed out, especially if mixtures camphorsulfonic acid and of a excipient selected from the group of arginine, meglumine, ornithine, and carnitine or mixtures thereof are added in equimolar amounts to the concentrated liquid protein composition, for example to solutions of mAbC, mAbD and mAbE, a particularly good viscosity reduction is achieved.

In further experiments, the potential of mixtures of any one of the tested cationic excipient in combination with camphorsulfonic acid to reduce the viscosity of highly concentrated antibody solutions (mAbC, mAbD & mAbE) was investigated. For each of these studies, mixtures of equimolar amounts of these excipients were added. Equimolar amounts of these excipients are preferred. Particularly good results were found here for concentrations of 150 mM of added excipients.

All model antibodies were formulated at a rather high concentrations of about 100 mg/ml, some of about 150mg/ml, especially of more than 200 mg/ml, and in particular of 220 mg/ml (mAbE) in either an acetate buffer at pH 5 or pH 5.5 or in phosphate buffer at pH 7.2. A chip-based (micro-electro-mechanical system) capillary rheometer, m-VROC^{™} (RheoSence, San Ramon, CA), was employed to measure the viscosity at 20 °C.

In all cases, the specific equimolar mixtures at a concentration of 150 mM of the cationic excipient show a significant reduction of the viscosity measured in the highly concentrated antibody solutions.

In further experiments, it has been found that a combination of camphorsulfonic acid and at least one of the cationic excipients mentioned above can significantly reduce the viscosity of antibody formulations when added to the liquid protein composition at a total concentration of 150 mM, respectively.

Also, in solutions in which mAbD is contained as a protein, the addition of each 150 mM camphorsulfonic acid and a cationic excipient as an excipient causes a significant reduction in viscosity.

In addition, as shown by the experiments carried out, various other combinations of the auxiliaries mentioned herein reduce the viscosity of high concentration antibody solutions. Therefore, the combinations of excipients mentioned here are not exhaustive, and there are other possible combinations that lead to corresponding results.

In this regard, the attempts to reduce the viscosity of the various liquid protein compositions have shown that, depending on the protein contained in the particular solution, different additives result in the best stabilizations and reductions in viscosities.

In this context the best formulation for the protein mAbC is a composition comprising phosphate buffer, Polysorbat 80, 75 mM camphorsulfonic acid, and 75 mM arginine dissolved in Milli-Q-Water and adjusted to pH 7.2.

For mAbE in turn the best formulation is a composition comprising acetate buffer, 0.1 g/L Polysorbate 80, 75mM camphorsulfonic acid and 75 mM arginine dissolved in Milli-Q-Water and adjusted to pH 5.5.

Therefore, disclosures consist in adding camphorsulfonic acid as an excipient in combination with cationic excipients as excipients selected from the group consisting of arginine, meglumine, ornithine, and carnitine either alone or in combination, to highly concentrated liquid protein compositions as described above for viscosity reduction. Particularly preferably, the addition of camphorsylfonic acid either in combination with meglumine or with ornithine, or with carnitine leads to good viscosity reductions. In another instance camphorsulfonic acid in combination with arginine as excipient leads also to especially good viscosity reductions as well in a solution comprising an acetate buffer at pH 5.0 as in a solution comprising a phosphate buffer at pH 7,2.

The formulation of solution preparations and freeze drying can be carried out by the methods as described above.

In summary, in highly concentrated liquid protein compositions the viscosity as critical physiological property can be advantageously reduced by addition of the excipient camphorsulfonic acid. Especially good viscosity reducing effects are achievable if camphorsulfonic is combined with at least one cationic excipient selected from the group arginine, meglumine, ornithine, carnitine. Thus, especially good viscosity reducing effects are achieved by the addition of the combinations of: meglumine with camphorsulfonic acid, ornithine with camphorsulfonic acid, carnitine with camphorsulfonic acid and arginine with camphorsulfonic acid.

In one aspect the present invention provides a method for reducing the viscosity of a liquid formulation comprising a pharmaceutically active protein in a concentration in the range of at least 50 mg/ml up to 300 mg/ml, comprising the step of combining the protein solution with at least camphorsulfonic acid and at least a cationic

excipient selected from the group meglumine, L-ornithine, and L-carnitine.

In another aspect the present invention provides a method as mentioned above, wherein the therapeutic protein is selected from the group of antibodies, antibody fragments, minibody, a nanobody, a modified antibody, antibody-like molecule and fusion protein.

In another aspect the present invention provides a method as mentioned above, wherein viscosity of the formulation is reduced by at least 12%.

In another aspect the present invention provides a method as mentioned above, wherein viscosity of the formulation is reduced by at least 50%.

In another aspect the present invention provides a liquid pharmaceutical formulation obtainable by the method as mentioned above having a reduced viscosity compared to an identical formulation without the excipient combinations.

In another instance, the disclosure provides a liquid pharmaceutical formulation as mentioned above, comprising a therapeutic protein in a concentration of at least 90 mg/ml up to 250 mg/ml and camphorsulfonic acid as a viscosity reducing excipient.

In another instance, the disclosure provides a liquid pharmaceutical formulation as mentioned above, wherein the concentration of the excipient is less than about 500 mM, especially less than 200 mM.

In another instance, the disclosure provides a liquid pharmaceutical formulation as mentioned above, having a pH in the range between about 4.5 to about 8.0 and comprising a buffer.

In another instance, the disclosure provides a liquid pharmaceutical formulation as mentioned above, having a pH in the range between about 4.5 to about 7.5 and comprising a buffer.

In another instance, the disclosure provides a liquid pharmaceutical formulation as mentioned above, having a pH of about 5 to about 7.2 and comprising a buffer.

In another instance, the disclosure provides a liquid pharmaceutical formulation as mentioned above, comprising a phosphate or acetate buffer.

In another instance, the disclosure provides a liquid pharmaceutical formulation as mentioned above, comprising a stabilizer.

In another instance, the disclosure provides a liquid pharmaceutical formulation as mentioned above, comprising sugar or a surfactant as stabilizer.

In another instance, the disclosure provides a liquid pharmaceutical formulation as mentioned above, comprising sucrose as a stabilizer.

In another instance, the disclosure provides a liquid pharmaceutical formulation as mentioned above, comprising polysorbate or Poloxamer 80 as stabilizer.

In another instance, the disclosure provides a method of preparing a lyophilized powder comprising the step of lyophilizing the pharmaceutical composition as mentioned above.

In another instance, the disclosure provides a lyophilized powder as mentioned above comprising a therapeutic protein and camphorsulfonic acid, wherein camphorsulfonic acid or a combination of camphorsulfonic acid with a cationic excipient is present in an amount sufficient to upon reconstitution vield to a concentration of less than 500 mM, preferably less than 200 mM.

In another instance, the disclosure provides a method for reconstituting a lyophilized powder as mentioned above, comprising the step of adding a sterile aqueous diluent.

In another instance, the disclosure provides the methods as mentioned above, wherein the therapeutic protein is selected from the group of antibodies, antibody fragments, minibody, a nanobody, a modified antibody, antibody-like molecule and fusion protein.

In another instance, the disclosure provides the pharmaceutical formulation or pharmaceutical composition as mentioned above, wherein the therapeutic protein is selected from the group of antibodies, antibody fragments, minibody, a nanobody, a modified antibody, antibody-like molecule and fusion protein.

In another instance, the disclosure provides the lyophilized powder as mentioned above, wherein the therapeutic protein is selected from the group of antibodies, antibody fragments, minibody, a nanobody, a modified antibody, antibody-like molecule and fusion protein.

In another instance, the disclosure provides a kit comprising a pharmaceutical formulation or a lyophilized powder as mentioned above.

In another instance, the disclosure provides a kit as mentioned above, comprising freeze-dried or spray-dried preparations of a pharmaceutical composition, obtained by a method as mentioned above which can be made into solution preparations prior to use.

In another instance, the disclosure provides a kit as mentioned above, comprising ready-to-use freeze-dried or spray-dried formulations sitting in a 96-well plate.

In another instance, the disclosure provides a kit as mentioned above for administration to patients, including a container, syringe and/or other administration device with or without needles, infusion pumps, iet iniectors, pen devices, transdermal injectors, or other needle-free injector and instructions.

Furthermore, it has been found that the excipients and excipient combinations as mentioned above are beneficial in the bioprocess. Excipients and excipient combinations were found to reduce the backpressure on chromatography columns and allow for larger flow rates. This leads to less shear forces straining the proteins in the solution and therefore, aggregation will be reduced. Altogether a higher yield can be obtained. Beyond this, when the process can be run using higher flow rates, the process time will be reduced significantly.

In bioprocesses as meant here, the addition of these excipients, which are found to serve as viscosity-reducing additives, lead to an improved process economy, in that on the one hand the yield of intact protein can be improved, and on the other hand the duration of the process can be reduced.

In the present invention the foundation of the experiments conducted is laid by Amicon^{®} centrifugal filtration studies. These experiments resemble any process step, where a solution is passed through a filter membrane by centrifugal force. The resistance to the centrifugal force is dependent on the properties of the filter, which is assumed to be constant within the context of this experiments, and the viscosity of the solution. Excipients and excipient combinations that provide the most favorable solutions are selected for more complex experiments using stirred cells.

In experiments using Amicon^{®} stirred cells nitrogen gas is used to apply pressure to the solution that is being pressed through a filter. The resistance of the pressure is dependent on the properties of the filter, which is assumed to be constant within the context of this experiments, and the viscosity of the solution. The excipient combination that gives most favorable results is used in an experiment using a laboratory scale tangential flow filtration (TFF) system.

These two experiments highlight the beneficial effects of viscosity reducing agents during dead-end filtration approaches. Additionally, it becomes clear that in technical approaches, where a solution is passed through a filter or medium, like a gel bed, by a force applied by back-end pressure, e.g. during chromatographic purifications, the disclosed viscosity reducing excipients have beneficial effects. While said filtration steps are mainly used in the downstream process, viscosity reducing excipients can also be beneficial in the upstream process. When protein concentrations elevate to levels where they cause viscosity, with the described negative effects of pressure limitations and shear forces when the solution is passed through a tubing or a filter to remove cellular material and debris the presented invention will obviously have beneficial effects. To measure process efficiency in tangential flow filtration, where in contrast to previously used method the majority of the field flow travels tangentially across the surface of the filter, rather than passing through the filter, a laboratory scale TFF system was used. While the filtration principle is different than in the methods described previously, also here the filtration efficiency depends on the resistance of the membrane, which also here remains constant, and the solution viscosity, which is modified by the invention. Filtration methods are typical unit operation used to exchange a formulation buffer or to bring the concentration of a biomolecule to the desired level. The stirred cells used herein are representation of dead-end filters, where a feed is passed through a filtering material that withholds larger molecules on top of the material releasing the filtrate on the other end of the device.

A frequent method to exchange buffers and to concentrate proteins is tangential flow filtration, where in contrast to previously used methods the majority of the field flow travels tangentially across the surface of the filter, rather than passing through the filter. Like when stirred cells are used in tangential flow filtration the large molecules are separated from smaller molecules by passing said smaller molecules through a suitable filter material. In contrast to stirred cells, which represent one form of dead end filtration, in tangential flow filtration the flow geometry of the feed is different to avoid the formation of a filter cake and allowing for a continuous process. When stirred cells are used the formation of a filter cake is likewise prevented by the use of a stirring device. Therefore, the stirred cells closely resemble a tangential flow filtration device in spite of the differences in filter geometry. The efficiency of both methods is critically depending on the membrane resistance. Also, a high viscosity is known to reduce the flux rate that can be used and therefore increasing processing time resulting in higher production costs. It is therefore expected that a reduced viscosity allows for a more efficient filtration process while shear forces remain low yielding to a higher protein concentration in the filtrate. This is highlighted by the work of Hung et al. who state: "During production of concentrated monoclonal antibody formulations by tangential flow ultrafiltration (TFF), high viscosities and aggregation often cause extensive membrane fouling, flux decay and low product yields" (Journal of Membrane Science Volume 508, 15 June 2016, Pages 113-126)

Experiments have shown that camphorsulfonic acid is suitable to improve bioprocess economics as described before. Furthermore, camphorsulfonic acid in combination with cationic excipients as excipients selected from the group consisting of arginine, meglumine, ornithine, and carnithine and two of said excipients improve bioprocess economics. Especially combinations in various ratios depending on the protein solutions improve bioprocess economics as described.

Therefore, in another instance, the disclosure provides for a method for reducing the viscosity of a liquid composition in bioprocess, comprising a protein in a concentration in the range of at least 50 mg/ml up to 300 mg/ml, comprising the step of combining the liquid composition with at least camphorsulfonic acid as an excipient in a concentration with a viscosity-reducing effect, preferably with at least one cationic excipient, more preferably selected from the list comprising arginine, meglumine, ornithine and carnitine.

Another aspect of the present invention is the use of the method for reducing the viscosity of a liquid composition as described in the claims in a bioprocess.

According to the present invention all parameters mentioned above; e.g. concentrations of the excipients, concentrations the protein, rations of the excipients, further elements of the composition such as buffers or stabilizers, pH values, viscosity reductions, specifications of the protein, molecular weight of the protein also apply to the use in bioprocess.

For the use in bioprocess, camphorsulfonic acid is use in a concentration of 75 - 500 mM, more preferred 75 - 150 mM, most preferred 75 mM or 150 mM. When camphorsulfonic acid is used in combination a cation selected from the group arginine, carnitine, meglumine and ornithine the concentration of each excipient is of 75 - 500 mM, more preferred 75 - 150 mM, most preferred 75 mM or 150 mM. When camphorsulfonic acid is used in combination with a cation selected from the group arginine, carnitine, meglumine and ornithine, the ratio is in the range of 1: 3 to 3: 1, more preferred 1:2 to 2:1, most preferred 1:1. For example, a combination of 25 mM carnithine with 50 mM camphorsulfonic acid yields to an excipient concentration of 75 mM in solution of a 1 : 2 mixture of these excipients.

Depending on the protein solution and the bioprocess carried out, different buffer systems may be used as buffers. Acetates, like ammonium acetate, or sodium acetate, carbonates like ammonium bicarbonate or sodium bicarbonate, or phosphates, like sodium phosphate or Tris-phosphate may be used here, depending on the conditions during the bioprocess.

Another disclosure relates to a method for reducing the viscosity of a liquid composition in a bioprocess as mentioned above, wherein the permeate flux of the liquid composition in a filtration step is increased compared to an identical liquid composition not comprising camphorsulfonic acid or compared to an identical liquid composition not comprising camphorsulfonic acid and at least one cationic excipient, preferably selected from a group consisting of arginine, meglumine, ornithine, and carnitine

Another aspect of the present invention is the use of the method in a bioprocess as claimed wherein the permeate flux of the liquid composition in a filtration step is increased compared to an identical liquid composition not comprising camphorsulfonic acid and at least one cationic excipient , selected from a group consisting of meglumine, ornithine, and carnitine

Increase of permeate flux means a percentage increase of at least 2%, preferably at least 5%, more preferably at least 10%, most preferred 10% to 100%.

Another disclosure relates a method for reducing the viscosity of a liquid composition in a bioprocess as mentioned above, wherein the protein recovery after buffer exchange and volume reduction in filters is increased compared to an identical liquid composition not comprising camphorsulfonic acid or compared to an identical liquid composition not comprising camphorsulfonic acid and at least one cationic excipient, preferably selected from a group consisting of arginine, meglumine, ornithine, and carnitine.

Another aspect of the present invention is the use of the method in a bioprocess as claimed, wherein the protein recovery after buffer exchange and volume reduction in filters is increased compared to an identical liquid composition not comprising camphorsulfonic acid and at least one cationic excipient, selected from a group consisting of meglumine, ornithine, and carnitine Increase of protein recovery after buffer exchange and volume reduction in filters means a percentage increase of protein recovery of at least 1%, preferably at least 2%, more preferably at least 5%, most preferred 5% to 20%.

Another aspect of the present disclosure is to provide a method for reducing the viscosity of a liquid composition in a bioprocess as mentioned above, wherein the process time for a filtration step, preferably a filtration step wherein the protein is concentrated, is reduced compared to an identical liquid composition not comprising camphorsulfonic acid or compared to an identical liquid composition not comprising camphorsulfonic acid and at least one cationic excipient, preferably selected from a group consisting of arginine, meglumine, ornithine, and carnitine.

Another aspect of the present disclosure is the use of the method in a bioprocess as mentioned above, wherein the process time for a filtration step, preferably a filtration step wherein the protein is concentrated, is reduced compared to an identical liquid composition not comprising camphorsulfonic acid or compared to an identical liquid composition not comprising camphorsulfonic acid and at least one cationic excipient, preferably selected from a group consisting of arginine, meglumine, ornithine, and carnitine.

Reduction of process time for a filtration step means a percentage reduction of at least 5%, preferably at least 10%, more preferably at least 25%, most preferred 25% to 100%.

In a particular instance, the filtration step is a tangential flow filtration (TFF).

Other aspects of the present disclosure are to provide kits for carrying out the methods described herein. In general, the kits for practice are adapted to the methods of the invention and the forms of the parts depend on their intended functions.

Typically, the kits are packaged and include vessels containing reagents, the solution volumes of which will vary based on the amount of preparations for which the kit is rated. The vessels will generally include one or more reagents useful in carrying out the method of the present invention. In certain instances, the kit is a compartmentalized kit, that is, the kit includes reagents contained in the same or separate vessels. Examples of vessels include, but are not limited to, small glass containers, plastic containers, or strips of plastic paper. These or other small vessels allow the efficient transfer of reagents from one compartment to another.

Such containers can include a container which will accept the test sample, a container which contains the protein or proteinic solution of the disclosure, containers which contain materials, containers which contain wash reagents, and/or containers which contain reagents useful in the application of the kit. The kit can include sources and concentrations of the polypeptides described herein. For larger scale applications, the kits will generally include similar reagents and solutions, but in larger quantity.

The disclosure also provides a kit comprising a liquid protein formulation of the invention, and instructions for its administration, optionally with a container, syringe and/or other administration device. The disclosure further provides a kit comprising a lyophilized protein formulation of the invention, optionally in a container, and instructions for its reconstitution and administration, optionally with a vial of sterile diluent, and optionally with a syringe or other administration device. Exemplary containers include vials, tubes, bottles, single or multi-chambered prefilled syringes, or cartridges, but also a 96-well plate comprising ready-to-use freeze-dried or spray-dried formulations sitting in the wells. Exemplary administration devices include syringes, with or without needles, infusion pumps, jet injectors, pen devices, transdermal injectors, or other needle-free injectors.

The kits will also typically include instructions for use. The instructions will generally be suitable to enable an end user to carry out the desired preparation or assay. The instructions will generally be in a tangible expression, e. g., describing the reagent concentration for at least one preparation or assay, parameters such as the relative amount of reagent and sample to be admixed, maintenance or incubation time periods for reagent/sample admixtures, temperature requirements or preferences, and the like. The instructions may be printed on the outer or inner packaging of the kit, in a brochure, a card, or other paper within the kit, and/or on the outer surface of the containers or vessels included in the kit.

All examples in the present disclosure are provided as non-limiting examples.

The formulation of solution preparations and freeze drying can be carried out by the methods as known to the skilled person.

The present description enables one of ordinary skill in the art to practice the present invention comprehensively. Even without further comments, it is therefore assumed that a person of ordinary skill in the art will be able to utilise the above description in the broadest scope.

For better understanding and in order to illustrate the invention, examples are presented below which are within the scope of protection of the present invention. These examples also serve to illustrate possible variants.

Furthermore, it goes without saying to one of ordinary skill in the art that, both in the examples given and also in the remainder of the description, the component amounts present in the compositions always only add up to 100% by weight or mol%, based on the composition as a whole, and cannot exceed this percentage, even if higher values could arise from the per cent ranges indicated. Unless indicated otherwise, % data are therefore % by weight or mol%, with the exception of ratios, which are shown in volume data.

The invention further provides a method for reducing the viscosity of a liquid composition comprising a protein, whereas the protein is Infliximab. In the present invention Infliximab is referred to by the abbreviation "mAbC". Preferably the Infliximab concentration in the pharmaceutical formulations is between 122 mg/ml and 185 mg/ml.

Infliximab (REMICADE^{®}) developed by Janssen Biotech, Inc. and its biosimilar drugs (FUXABI^{®}) developed by Biogen, (Inflectra) by Celltrion, is used in treatment of rheumatoid arthritis, adult ulcerative colitis, plaque psoriasis, psoriatic arthritis, ankylosing spondylitis, adult & pediatric Crohn's disease (Dose/Dosage: 5 mg/kg). Infliximab is a mAb against tumor necrosis factor alpha (TNF-a) used to treat autoimmune diseases. Infliximab neutralizes the biological activity of TNFa by binding with high affinity to the soluble and transmembrane forms of TNFa and inhibits binding ofTNFa with its receptors. It is marketed under the trade name REMICADE^{®} by Janssen Global Services, LLC ("Janssen") in the U.S., Mitsubishi Tanabe Pharma in Japan, Xian Janssen in China, and Merck Sharp & Dohme ("MSD"); elsewhere. In some embodiments, the formulations contain a biosimilar of REMICADE^{®}, such as REMSIMA^{™} or INFLECTRA^{™}. Both REMSIMA^{™}, developed by Celltrion, Inc. ("Celltrion"), and INFLECTRA ^{™}, developed by Hospira Inc., UK. Infliximab is currently administered via iv infusion at doses ranging from about 3 mg/kg to about 10 mg/kg.

The invention further provides a method for reducing the viscosity of a liquid composition comprising a protein, whereas the protein is Evolocumab. In the present invention Evolocumab is referred to by the abbreviation "mAbD". Preferably the Evolocumab concentration in the pharmaceutical formulations is between 163 mg/ml and 204 mg/ml.

Evolocumab (REPATHA^{®}) developed by Amgen is used in treatment of HeFH, CVD, reducing of low density lipoprotein cholesterol (LDL-C) by targeting PCSK9 (proprotein convertase subtilisin kexin type 9) (Dose/Dosage: 420 mg monthly).

The invention further provides a method for reducing the viscosity of a liquid composition comprising a protein, whereas the protein is Reslizumab. In the present invention Reslizumab is referred to by the abbreviation "mAbE". Preferably the Reslizumab concentration in the pharmaceutical formulations is between 178 mg/ml and 224 mg/ml.

Reslizumab (CINQAIR^{®}) was developed by Teva Pharmaceuticals and is used in severe asthma attacks (exacerbations) (Dose/Dosage: 3 mg/kg).

### Examples:

In the Examples, the following abbreviations are used for the mAbs:
mAbC: Infliximab
mAbD: Evolocumab
mAbE: Reslizumab

### Example 1

Effect of meglumine, L-ornithine, L-carnitine and camphorsulfonic acid on highly concentrated protein solutions:
Example 1a) shows that L-arginine, L-carnitine and camphorsulfonic acid, but not L-ornithine and meglumine reduce the viscosity of mAbC at 98 mg/ml and 148 mg/ml respectively.
Example 1b) shows that meglumine, L-ornithine, L-carnitine and camphorsulfonic acid reduce the viscosity of mAbD at 170 mg/ml and 190 mg/ml.
Example 1c) shows that meglumine, L-ornithine, L-carnitine and camphorsulfonic acid reduce the viscosity of mAbE at 179 mg/ml and 223 mg/ml. Control samples in all cases is the market formulation of the respective mAb without the viscosity reducing excipient.

### Example 1a

Viscosity reducing effect of L-arginine, L-carnitine and camphorsulfonic acid, but not L-ornithine and meglumine reduce the viscosity of mAbC formulated in phosphate buffer at pH 7.2.

### Buffer Preparation

5 mM Phosphate buffer was prepared by appropriately mixing sodium dihydrogenphosphate and di-sodium hydrogenphosphate to yield a pH of 7.2 and dissolving the said mixture in ultrapure water. The said ratio was determined using the Henderson-Hasselbalch equation. pH was adjusted using HCI and NaOH if necessary.

50 mg/ml Sucrose and 0.05 mg/ml Polysorbate 80 were added as stabilizers.

### Sample Preparation

Excipient solutions of 150 mM L-ornithine, L-arginine, L-carnitine, meglumine, and camphorsulfonic acid were prepared in Phosphate buffer pH 7.2, respectively. The pH was adjusted using HCL or NaOH, if necessary.

A concentrated mAb solution containing the desired excipients was prepared using centrifugal filters (Amicon, 30 kDA MWCO) to exchange the original buffer with a buffer containing the relevant excipients and to reduce the volume of the solution. The protein was subsequently diluted to 98 mg/ml and 148 mg/ml respectively.

### Viscosity Measurements

The mVROC^{™} Technology (Rheo Sense, San Ramon, California USA) was used for viscosity measurements.

Measurements were performed at 20 °C using a 500 µl syringe and a shear rate of 3000 s⁻¹. A volume of 200 µl was used. All samples were measured as triplicates.

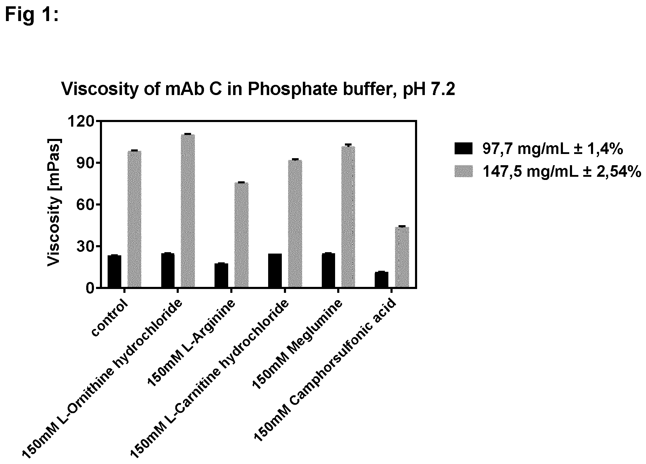

Figure 1 shows the viscosity reducing effect of meglumine, L-ornithine, L-carnitine, arginine and camphorsulfonic acid of mAbC formulated in phosphate buffer pH 7.2 (Example 1a).

### Example 1b

Meglumine, L-ornithine, L-carnitine and camphorsulfonic acid reduce the viscosity of mAbD at 170 mg/ml and 190 mg/ml.

### Buffer Preparation

20 mM Acetate buffer was prepared by mixing 1.2 mg/ml glacial acetic acid with ultrapure water. pH was adjusted to 5.0 using HCI and NaOH if necessary.

0.1 mg/ml Polysorbate 80 was added as stabilizer

### Sample Preparation

Excipient solutions of 150 mM L-ornithine, L-arginine, L-carnitine, meglumine, and camphorsulfonic acid were prepared in acetate buffer pH 5.0, respectively. The pH was adjusted using HCL or NaOH, if necessary.

A concentrated mAb solution containing the desired excipients was prepared using centrifugal filters (Amicon, 30 kDA MWCO) to exchange the original buffer with a buffer containing the relevant excipients and to reduce the volume of the solution. The protein was subsequently diluted to 169 mg/ml and 190 mg/ml respectively.

### Viscosity Measurements

The mVROC^{™} Technology (Rheo Sense, San Ramon, California USA) was used for viscosity measurements.

Measurements were performed at 20 °C using a 500 µl syringe and a shear rate of 3000 s⁻¹ for protein solutions at 169 mg/ml and of 2000 s⁻¹ for protein solutions at 190 mg/ml. A volume of 200 µl was used. All samples were measured as triplicates.

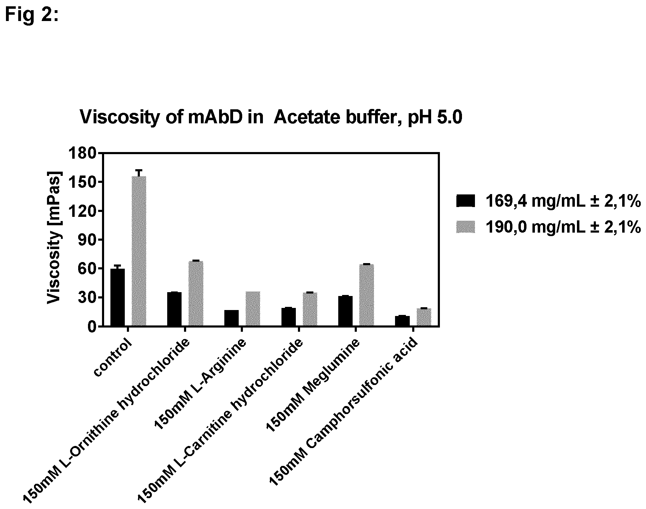

Figure 2 shows the viscosity reducing effect of meglumine, L-ornithine, L-carnitine, arginine and camphorsulfonic acid of mAbD formulated in acetate buffer pH 5.0 (Example 1b).

### Example 1c

Meglumine, L-ornithine, L-carnitine and camphorsulfonic acid reduce the viscosity of mAbE at 179 mg/ml and 223 mg/ml.

### Buffer Preparation

20 mM acetate buffer was prepared by mixing Sodium acetate and glacial acetic acid with ultrapure water in a ratio yielding to a buffer solution at pH 5.5. The ratio of Sodium acetate and glacial acetic acid was calculated using the Henderson-Hasselbalch equation. pH was adjusted using HCI and NaOH if necessary. 70 mg/ml Sucrose were added as a stabilizer

### Sample Preparation

Excipient solutions of 150 mM L-ornithine, L-arginine, L-carnitine, meglumine, and camphorsulfonic acid were prepared in acetate buffer pH 5.5, respectively. The pH was adjusted using HCL or NaOH, if necessary.

A concentrated mAb solution containing the desired excipients was prepared using centrifugal filters (Amicon, 30 kDA MWCO) to exchange the original buffer with a buffer containing the relevant excipients and to reduce the volume of the solution. The protein was subsequently diluted to 179 mg/ml and 223 mg/ml respectively.

### Viscosity Measurements

The mVROC^{™} Technology (Rheo Sense, San Ramon, California, USA) was used for viscosity measurements.

Measurements were performed at 20 °C using a 500 µl syringe and a shear rate of 3000 s⁻¹. A volume of 200 µl was used. All samples were measured as triplicates.

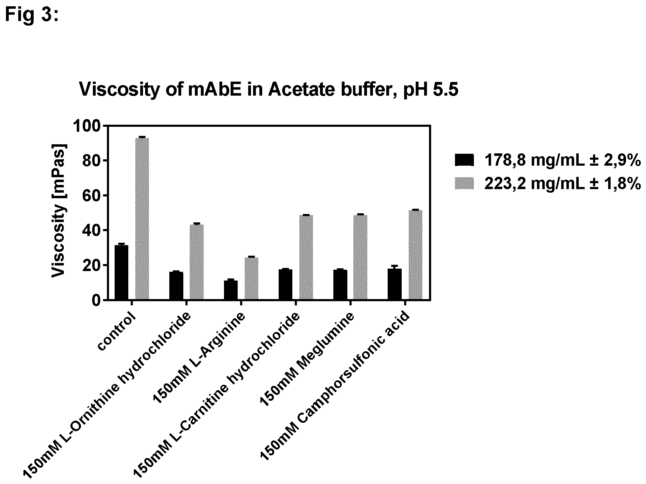

Figure 3 shows the viscosity reducing effect of meglumine, L-ornithine, L-carnitine, arginine and camphorsulfonic acid of mAbE formulated in acetate buffer pH 5.5 (Example 1c).

### Example 2

Effect of the combinations of L-arginine, L-carnitine, L-ornithine, and meglumine with camphorsulfonic acid.
Example 2a) shows that meglumine, L-ornithine, L-carnitine when combined with camphorsulfonic acid reduce the viscosity of mAbC at 98 mg/ml and 148 mg/ml respectively.
Example 2b) shows that meglumine, L-ornithine, L-carnitine when combined with camphorsulfonic acid reduce the viscosity of mAbD at 170 mg/ml and 190 mg/ml.
Example 2c) shows meglumine, L-ornithine, L-carnitine when combined with camphorsulfonic acid reduce the viscosity of mAbE at 179 mg/ml and 223 mg/ml.
Example 2d) shows synergistic viscosity reduction by combining L-arginine with camphorsulfonic acid on mAbC formulated in phosphate buffer pH 7.2.

### Example 2a

### Buffer Preparation

5 mM Phosphate buffer was prepared by appropriately mixing sodium dihydrogenphosphate and di-sodium hydrogenphosphate to yield a pH of 7.2 and dissolving the said mixture in ultrapure water. The said ratio was determined using the Henderson-Hasselbalch equation. pH was adjusted using HCI and NaOH if necessary. 50 mg/ml Sucrose and 0.05 mg/ml Polysorbate 80 were added as stabilizers.

### Sample Preparation

Excipient solutions of 75 mM L-ornithine hydrochloride, L-arginine, L-carnitine, meglumine, supplemented with additional 75 mM camphorsulfonic acid were prepared in phosphate buffer pH 7.2, respectively. The pH was adjusted using HCL or NaOH, if necessary.

A concentrated mAb solution containing the desired excipients was prepared using centrifugal filters (Amicon, 30 kDA MWCO) to exchange the original buffer with a buffer containing the relevant excipients and to reduce the volume of the solution. The protein was subsequently diluted to 98 mg/ml and 148 mg/ml respectively.

### Viscosity Measurements

The mVROC^{™} Technology (Rheo Sense, San Ramon, California USA) was used for viscosity measurements.

Measurements were performed at 20 °C using a 500 µl syringe and a shear rate of 3000 s⁻¹. A volume of 200 µl was used. All samples were measured as triplicates.

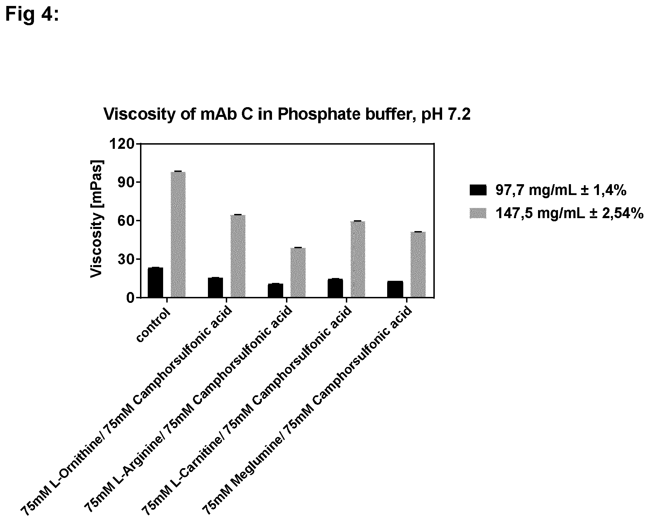

Figure 4 shows the viscosity reducing effect of meglumine, L-ornithine, L-carnitine when combined with camphorsulfonic acid of mAbC formulated in phosphate buffer pH 7.2 (Example 2a).

### Example 2b

### Buffer Preparation

20 mM Acetate buffer was prepared by mixing 1,2 mg/ml glacial acetic acid with ultrapure water. pH was adjusted to 5.0 using HCI and NaOH if necessary.

0.1 mg/ml Polysorbate 80 was added as stabilizer

### Sample Preparation

Excipient solutions of 75 mM L-ornithine hydrochloride, L-arginine, L-carnitine, meglumine, supplemented with additional 75 mM camphorsulfonic acid were prepared in acetate buffer pH 5.0, respectively. The pH was adjusted using HCL or NaOH, if necessary.

A concentrated mAb solution containing the desired excipients was prepared using centrifugal filters (Amicon, 30 kDA MWCO) to exchange the original buffer with a buffer containing the relevant excipients and to reduce the volume of the solution. The protein was subsequently diluted to 169 mg/ml and 190 mg/ml respectively.

### Viscosity Measurements

The mVROC^{™} Technology (Rheo Sense, San Ramon, California USA) was used for viscosity measurements.

Measurements were performed at 20 °C using a 500 µl syringe and a shear rate of 3000 s⁻¹ for protein solutions at 169 mg/ml and of 2000 s⁻¹ for protein solutions at 190 mg/ml s⁻¹. A volume of 200 µl was used. All samples were measured as triplicates.

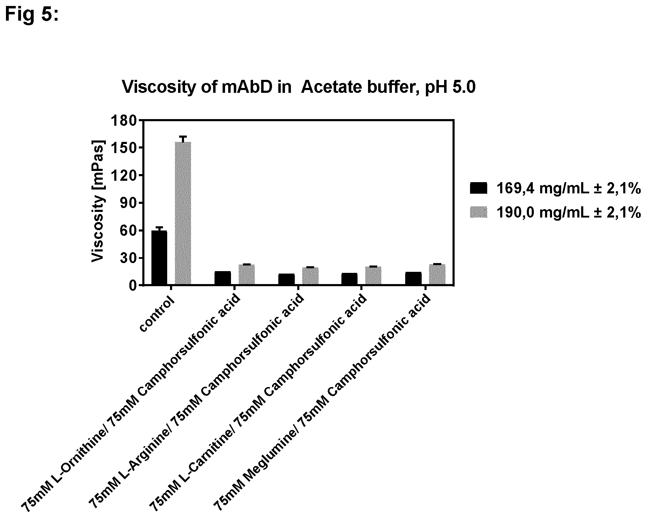

Figure 5 shows the viscosity reducing effect of meglumine, L-ornithine, L-carnitine when combined with camphorsulfonic acid of mAbD formulated in acetate buffer pH 5.0 (Example 2b).

### Example 2c

### Buffer Preparation

20 mM acetate buffer was prepared by mixing Sodium acetate and glacial acetic acid with ultrapure water in a ratio yielding to a buffer solution at pH 5.5. The ratio of Sodium acetate and glacial acetic acid was calculated using the Henderson-Hasselbalch equation. pH was adjusted using HCI and NaOH if necessary.

70 mg/ml Sucrose were added as a stabilizer

### Sample Preparation

Excipient solutions of 75 mM L-ornithine, L-arginine, L-carnitine, meglumine, supplemented with additional 75 mM camphorsulfonic acid were prepared in acetate buffer pH 5.5, respectively. The pH was adjusted using HCL or NaOH, if necessary.

A concentrated mAb solution containing the desired excipients was prepared using centrifugal filters (Amicon, 30 kDA MWCO) to exchange the original buffer with a buffer containing the relevant excipients and to reduce the volume of the solution. The protein was subsequently diluted to 179 mg/ml and 223 mg/ml respectively.

### Viscosity Measurements

The mVROC^{™} Technology (Rheo Sense, San Ramon, California USA) was used for viscosity measurements.

Measurements were performed at 20 °C using a 500 µl syringe and a shear rate of 3000 s⁻¹. A volume of 200 µl was used. All samples were measured as triplicates.

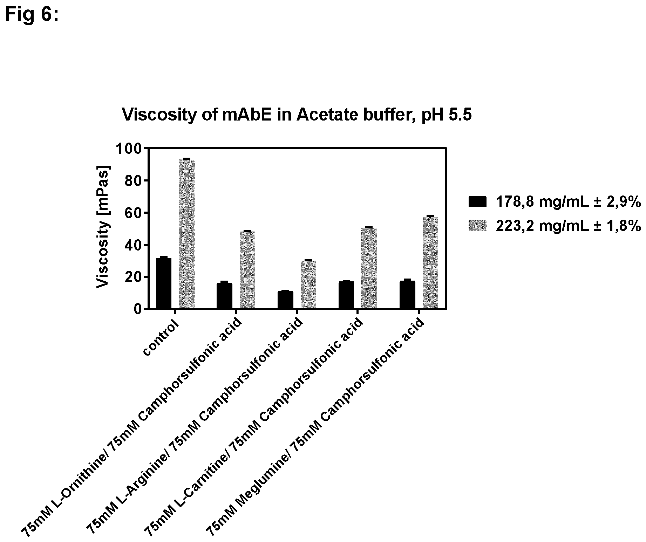

Figure 6 shows the viscosity reducing effect of meglumine, L-ornithine, L-carnitine when combined with camphorsulfonic acid of mAbE formulated in acetate buffer pH 5.5 (Example 2c).

### Example 2d

Buffer Preparation, Sample Preparation and Viscosity Measurements were performed according to example 1a) and 2a).

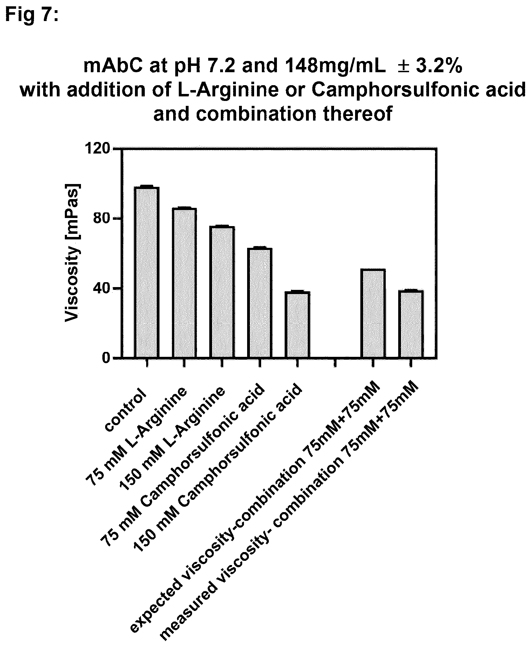

Figure 7 shows the synergistic viscosity reducing effect of L-arginine when combined with camphorsulfonic acid of mAbC formulated in phosphate buffer pH 7.2 (Example 2d). The 'expected viscosity-combination' corresponds to an assumed additive effect of each excipients, L-ornithine and L-arginine, at 75mM alone. The measured combination revealed an even lower viscosity proving a synergy between these two excipients on mAbC at 148 mg/mL.

### Example 3

Example 3 shows the effect of the camphorsulfonic acid and combinations thereof with L-ornithine or L-arginine on protein stability of mAbC.

### Buffer Preparation

5 mM Phosphate buffer was prepared by appropriately mixing sodium dihydrogenphosphate and di-sodium hydrogenphosphate to yield a pH of 7.2 and dissolving the said mixture in ultrapure water. The said ratio was determined using the Henderson-Hasselbalch equation. pH was adjusted using HCl and NaOH if necessary. 50 mg/ml Sucrose and 0.05 mg/ml Polysorbate 80 were added as stabilizers.

### Sample Preparation

An excipient solution containing 150 mM L-ornithine, L-arginine or camphorsulfonic acid was prepared in phosphate buffer pH 7.2. Also 75 mM L-ornithine hydrochloride or L-arginine supplemented with additional 75 mM camphorsulfonic acid were prepared in phosphate buffer pH 7.2, respectively. The pH was adjusted using HCL or NaOH, if necessary.

A concentrated mAbC solution containing the desired excipients was prepared using centrifugal filters (Amicon, 30 kDA MWCO) to exchange the original buffer with a buffer containing the relevant excipients and to reduce the volume of the solution. The protein was subsequently diluted to approximately 80 mg/ml.

Samples were sterilized using a syringe filter (Millex GV, 0,22µm, PVDF, Art.No.: SLGV013SL) and aliquoted in previously rinsed and sterilized crimp vials. Vials were crimped and stored at 40 °C/ 75% rH for 28 days.

### Monomer content analysis

Monomer content was determined by size-exclusion chromatography using an Aquity UPLC Protein BEH SEC column attached to an Agilent 1290 Infinity UHPLC System. Size separation was performed isocratic with a potassium salt eluent containing 10% organic solvent at 30 °C. As standard (100%) an unstressed sample of mAbC (1 mg/mL) was used. Samples were diluted to 1 mg/mL for analyses.

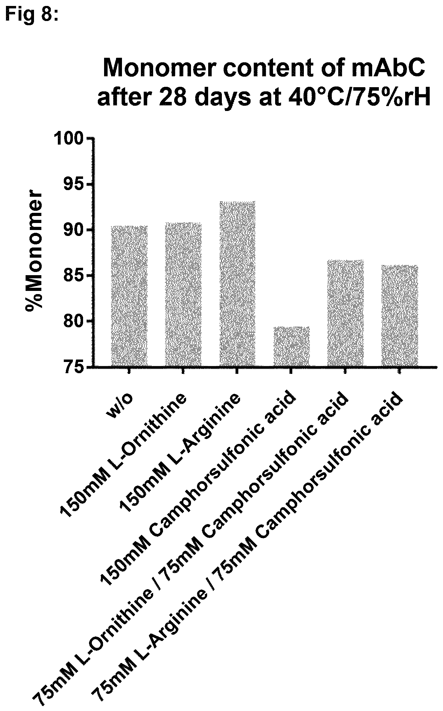

Figure 8 shows the residual monomer content of a solution comprising approximately 80 mg/mL mAbC in phosphate buffer pH 7.2 with or without excipients stored at 40 °C/ 75% rH for 28 days. Protein stability is lesser negatively affected when a combination of camphorsulfonic acid with L-arginine or L-ornithine is used instead when the acid is the only excipient.

### Example 4: Benefits for centrifugal filter units

### Buffer preparation

A 10 mM citrate buffer was prepared using citric acid monohydrate and dissolving it in ultrapure water. pH was adjusted using HCl and NaOH to 5.5 if necessary. 0,25 mg/mL Polysorbate 80 was added as stabilizer.

Excipient solutions of camphorsulfonic acid (CSAcid), ornithine(Orn or OM), arginine (Arg or AG), carnithine (Car) and meglumine (Meg or MG) were prepared in citrate buffer, pH 5.5 with a concentration of 150 mM. Combinations containing two of these excipients were prepared with a concentration of 75 mM for each or 150 mM for each.

### Sample Preparation

A Cetuximab solution containing approximately 14.7 mg/ml was used as starting material. Thereof, a sufficient volume was calculated to achieve a final concentration of more than 120 mg/ml in 500 µL sample assuming up to 20% sample loss.

### Protein concentration measurements

Protein concentration was determined using absorption spectroscopy applying Lambert-Beer's-Law. When excipients themselves have a strong absorbance at 280 nm a Bradford-Assay was used.

Concentrated protein solutions were diluted so that their expected concentration would lie between 0.3 and 1.0 mg/mL in the measurement.

For absorption spectroscopy the absorbance at 280 nm was measured at 280 nm using a BioSpectrometer^{®} kinetic (Eppendorf, Hamburg, Germany) with a protein extinction coefficient of A0.1%, 280nm=1.4.

For excipients that absorbed light at 280 nm protein concentration was determined using a Bradford-Assay. Therefore, a kit from Thermo ScientificTM (Thermo Fisher, Waltham, Massachusetts, USA) as well as a Cetuximab-Standard prepared by using absorption spectroscopy applying Lambert-Beer's-Law were used. Absorption was measured at 595 nm using a MultiskanTM Wellplatereader (Thermo Fisher, Waltham, Massachusetts, USA). Protein Concentrations were determined by an appropriate polynomial regression of a standard curve from 125 to 1500 µg/mL.

### Volume measurement

Permeate volume was measured using a volumetric flask of an appropriate size.

For Amicon^{®} centrifugal filter units the permeate was transferred to the volumetric flask after centrifugation. For Amicon^{®} stirred cell experiments, the permeate was directly collected in a such one.

Volume of concentrated protein solutions were measured using a Multipette^{®} E3X (Eppendorf, Hamburg, Germany) and Combitips advanced^{®} of an appropriate size.

### Buffer exchange and volume reduction

An Amicon^{®} centrifugal filter with a 30 kDa MWCO was used to exchange the original buffer with a buffer containing the relevant excipients and to reduce the volume of the solution.

Five diavolumes were used to exchange the original buffer with a buffer containing the relevant excipients.

To measure permeate flux, Amicon^{®} centrifugal filters were centrifuged at 2000 xg for 15 minutes and volume measured as described above (repeated four times and average calculated).

To achieve the final concentration, Amicon^{®} centrifugal filters were centrifuged in small timesteps and cumulated duration denoted upon reaching the 500 µL mark.

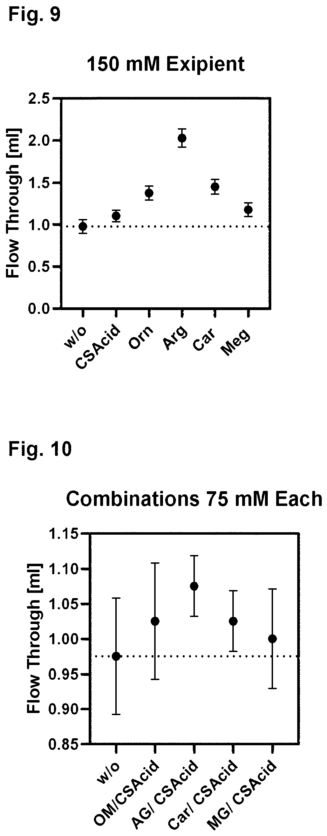

Fig. 9 to Fig. 11 highlight the process improvement indicated by an increased permeate flux.

It was found that 150 mM of each excipient increases the flow through under said experimental conditions. Using combinations comprising of 75 mM of CSAcid and a more cationic excipients also increases the flow through, in particular the combination AG/CSAcid.

It was found that when the concentration of the individual excipients is increased to 150 mM all tested combinations significantly increase the flow through.

Another aspect to characterize process benefits using Amicon^{®} centrifugal filters is the time required to reach a certain volume. This aspect is related to but differs from the average permeate flux since here the effect of the excipients at higher protein concentrations has a stronger influence on this parameter.

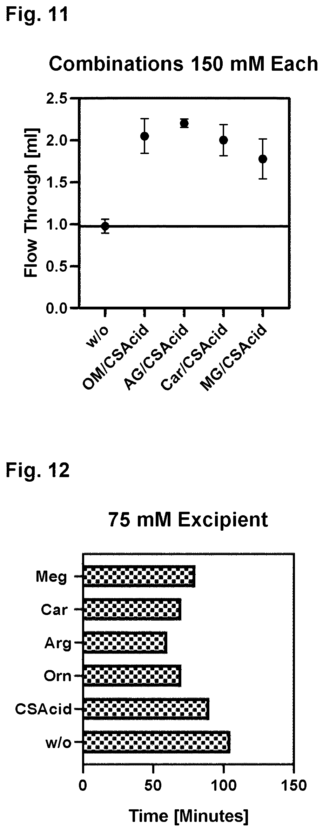

Fig. 12 to Fig. 14 show the decrease in process time that can be achieved by using 75 mM cationic or anionic excipient, respectively.

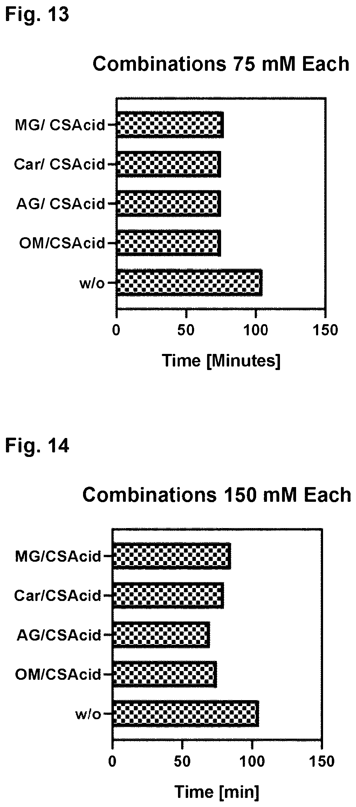

A reduced processing time can be observed for each of the listed excipients. Fig. 13 and 14 highlight the effect of excipient combinations on process time.

Each combination listed reduces processing time and has therefore a beneficial effect on process economics.

Another aspect that is critical for process economics other than processing time is process efficiency. This parameter can be assessed in this experimental setup by the recovery of protein. Recovery is defined as the fraction of protein that can be retrieved from the Amicon filter after the volume of the solution has been reduced to 0.5 ml.

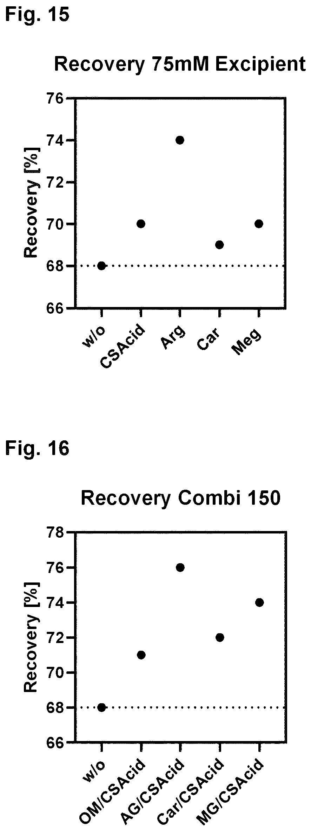

Fig. 15 and Fig. 16 show the effect of 75 mM cationic and/or anionic excipient respectively on protein recovery.

It was found that under all tested conditions the recovery is improved by the addition of the viscosity reducing excipients according to the present invention.

### Example 5: Benefits for stirred cells

Excipients and combinations that had a positive effect in processing with Amicon^{®} centrifugal filters were used in Amicon^{®} stirred Cell filtration. While Amicon^{®} spin column concentrators are driven by centrifugal force, stirred cells are operated by back pressure that is applied to the solution in form of nitrogen or air flow. This model system is frequently used to test processability of a solution and is a more closer model system compared to the previously used Amicon^{®} centrifugal filters.

### Buffer preparation

### See Example 1

### Sample preparation

The volume of antibody stock solution was calculated to yield at least 10 mL of a solution comprising 25 mg/mL cetuximab assuming a loss of up to 20%.

### Protein concentration measurements

### See Example 1

### Volume measurements

### See Example 1

### Buffer exchange and volume reduction

For stirred cell setup a model containing up to 50 mL was equipped witha Ultrafiltration disc filter with a NMWL of 30 kDa and an active membrane area of 13.4 cm².

The respective volume of cetuximab stock solution was filled in the stirred cell and respective buffer added to the 50 mL mark.

Five diavolumes were used to exchange the original buffer with a buffer containing the relevant excipient or combination.

To measure permeate flux, a pressure of 4 bar was applied on the Amicon^{®} stirred cell at a mixing speed of 200 rpm (using magnetic stirrer plate) for 30 minutes (four times).

For final concentration time, the cell was filled again to 50 mL mark with the respective buffer and 4 bar pressure applied at 200 rpm stirrer speed. Upon reaching the 10 mL mark, duration was denoted, process stopped and volume as well as concentration of the resulting antibody solution measured as described above.

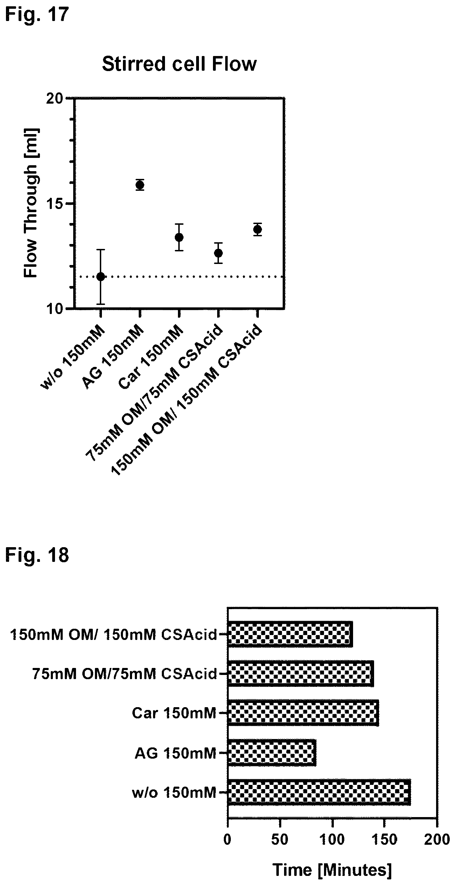

As in the previous section, the effect on mean permeate flux of the formulation is assessed first. Results are depicted in the following Fig. 17.

An improved permeate flux for each of the used excipients and excipient combinations was observed.

The next parameter to be analyzed is the process time. Here, the time until the volume of the formulation is reduced to 10 ml was measured. Results are depicted in Fig. 18.

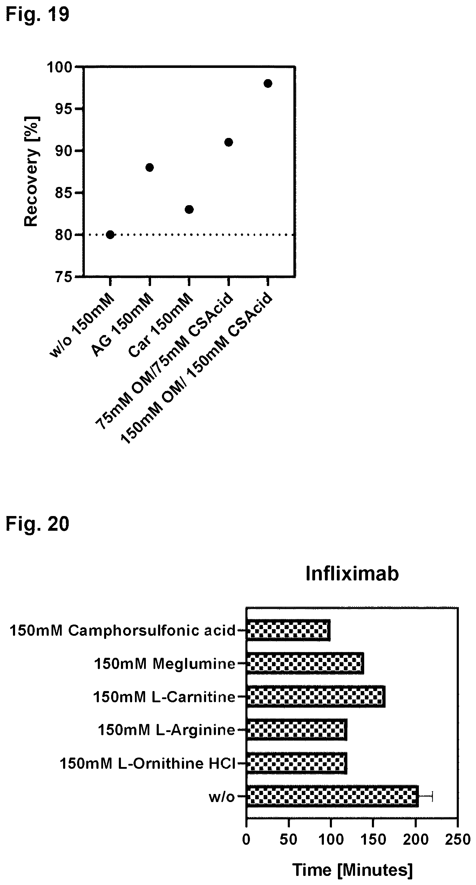

For each excipient and excipient combination used herein a reduction of processing time was observed. Finally, the process yield in form of protein recovery from the stirred cell was determined and is shown in Fig. 19.

An improved recovery for all tested conditions was observed.

### Example 5: Processing with Amicons: Infliximab

### Buffer Preparation

5 mM Phosphate buffer was prepared by appropriately mixing sodium dihydrogenphosphate and di-sodium hydrogenphosphate to yield a pH of 7.2 and dissolving the said mixture in ultrapure water. The said ratio was determined using the Henderson-Hasselbalch equation. pH was adjusted using HCl and NaOH if necessary.

50 mg/ml Sucrose and 0.05 mg/ml Polysorbate 80 were added as stabilizers.

### Sample Preparation

Excipient solutions with one compound were prepared if not stated otherwise at a concentration of 150 mM in Phosphate buffer pH 7.2. Folic acid was prepared in a 12 mM concentration. Thiamine pyrophosphate was prepared in a 75 mM concentration. The pH was adjusted using HCL or NaOH, if necessary.

In combination of two excipient, the respective compounds were prepared with a concentration of 75 mM each. Folic acid was used with 12 mM in combinatons.

A concentrated Infliximab solution containing the desired excipients was prepared using centrifugal filters (Amicon, 30 kDA MWCO) to exchange the original buffer with a buffer containing the relevant excipients and to reduce the volume of the solution.

As stock material a solution containing 10 mg/ml Infliximab was used and starting volume calculated so that a concentration of at least 160 mg/ml was achieved in 0.5 ml.

### Protein Concentration Measurements

### Protein Concentration was determined using a Bradford-Assay

Therefore a kit from Thermo Scientific^{™} (Thermo Fisher, Waltham, Massachusetts, USA) as well as a Infliximab-Standard prepared by using absorption spectroscopy applying Lambert-Beer's-Law were used. Absorption was measured at 595 nm using a Multiskan^{™} Wellplatereader (Thermo Fisher, Waltham, Massachusetts, USA). Protein Concentrations were determined by an appropriate polynomial regression of a standard curve from 125 to 1500 µg/mL.

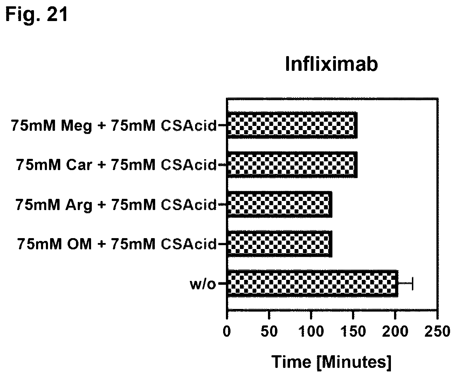

The processing time until a volume of approx. 0.5 ml are reached is depicted in Fig. 20 and Fig. 21.

With each excipient used in this study, the processing time can be reduced by at least 20 minutes, in some cases by up to 90 minutes. Time reduction that can be reached with combinations of excipients (75 mM each) are depicted in Fig. 21.

The use of the viscosity reducing excipient combinations reduces processing time for infliximab when Amicon filters are used as a model. As described previously, process benefits shown herein can be translate into an improved processability even when a more realistic model, e.g. TFF is used.

**It was found that**
- Viscosity of highly concentrated protein solution is reduced by L-arginine, L-ornithine, L-carnitine, meglumine, and camphorsulfonic acid.
- Viscosity of highly concentrated protein solution is reduced by camphorsulfonic acid in combination with L-arginine, L-ornithine, L-carnitine, meglumine.
- Viscosity is reduced stronger by a combination of L-arginine and camphorsulfonic acid than the theoretical reduction achieved by the sum of both excipients alone (synergistical combination).
- In relation to the viscosity reducing potential, protein stability is less negatively influenced when using camphorsulfonic acid in combination with L-arginine or L-ornithine compared to when camphorsulfonic acid is the only excipient.
- Permeate flux using Amicon^{®} centrifugal filters is increased by camphorsulfonic acid and combinations of camphorsulfonic acid with L-ornithine, L-arginine, L-carnitine and meglumine. Excipients and combinations increasing permeate flux in Amicon^{®} centrifugal filters can also increase this flux in Amicon^{®} stirred cells.
- Duration till final protein concentration was reached could be reduced by camphorsulfonic acid and combinations of camphorsulfonic acid with L-ornithine, L-arginine, L-carnitine and meglumine compared to a sample without the excipients.
- Recovery of antibody after buffer exchange and volume reduction could be increased by camphorsulfonic acid and combinations of camphorsulfonic acid with L-ornithine, L-arginine, L-carnitine and meglumine compared to a sample without the excipients. Excipients and combinations increasing protein recovery after buffer exchange and volume reduction in Amicon^{®} centrifugal filters can also increase this in Amicon^{®} stirred cells.

## Claims

1. A method for reducing the viscosity of a liquid composition comprising a protein in a concentration in the range of at least 50 mg/ml up to 300 mg/ml, comprising the step of combining the liquid composition with camphorsulfonic acid and at least one cationic excipient, wherein the at least one cationic excipient is selected from the list comprising meglumine, ornithine and carnitine in a concentration with a viscosity-reducing effect.

2. The method of claim 1, wherein the viscosity is reduced compared to an identical liquid composition not comprising camphorsulfonic acid and at least one cationic excipient selected from a group consisting of meglumine, ornithine and carnitine.

3. The method according to claim 1 or 2, wherein the liquid composition is a liquid pharmaceutical formulation, and the protein is a pharmaceutically active protein.

4. The method according to any one of the claims 1 to 3, wherein the protein is selected from the group of an antibody, an antibody fragment, a minibody, a nanobody, a modified antibody, an antibody-like molecule, an antibody-drug conjugate and a fusion protein.

5. The method according to any one of the claims 1 to 4, wherein the viscosity of the liquid composition is reduced by at least 12%, preferably by at least 50%.

6. The method according to any one of the claims 1 to 5, wherein the concentration of the protein is between 90 mg/ml to 250 mg/ml.

7. The method according to any one of the claims 1 to 6, wherein the concentration of camphorsulfonic acid is less than about 500 mM, especially less than 200 mM.

8. The method according to any one of the claims 1 to 7, wherein the pH of the liquid composition is in the range between about 3 to about 8 and comprising a buffer.

9. A liquid composition obtainable by the method of any one of the claims 1 to 8 having a reduced viscosity compared to an identical liquid composition without the excipient combinations.

10. Use of the method according to any one of the claims 1 to 8 in a bioprocess.

11. Use of the method according to claim 10, wherein the permeate flux of the liquid composition in a filtration step is increased compared to an identical liquid composition not comprising camphorsulfonic acid and at least one cationic excipient selected from a group consisting of meglumine, ornithine and carnitine.

12. Use of the method according to claims 10 or 11, wherein the protein recovery after buffer exchange and volume reduction in a filtration step is increased compared to an identical liquid composition not comprising camphorsulfonic acid and at least one cationic excipient selected from a group consisting of meglumine, ornithine and carnitine.

## Patentansprüche

1. Verfahren zur Reduzierung der Viskosität einer Flüssigzusammensetzung enthaltend ein Protein in einer Konzentration im Bereich von mindestens 50 mg/ml bis zu 300 mg/ml, umfassend den Schritt des Kombinierens der Flüssigzusammensetzung mit Camphersulfonsäure und mindestens einem kationischen Hilfsstoff, wobei der mindestens eine kationische Hilfsstoff ausgewählt ist aus der Liste enthaltend Meglumin, Ornithin und Carnitin in einer Konzentration mit viskositätsreduzierender Wirkung.

2. Verfahren des Anspruchs 1, wobei die Viskosität verglichen mit einer identischen Flüssigzusammensetzung, die Camphersulfonsäure und mindestens einen aus einer Gruppe bestehend aus Meglumin, Ornithin und Carnitin ausgewählten kationischen Hilfsstoff nicht enthält, reduziert ist.

3. Verfahren nach Anspruch 1 oder 2, wobei die Flüssigzusammensetzung eine pharmazeutische Flüssigformulierung ist und das Protein ein pharmazeutisch wirksames Protein ist.

4. Verfahren nach irgendeinem der Ansprüche 1 bis 3, wobei das Protein aus der Gruppe eines Antikörpers, eines Antikörperfragments, eines Minibodys, eines Nanobodys, eines modifizierten Antikörpers, eines antikörperähnlichen Moleküls, eines Antikörper-Medikament-Konjugats und eines Fusionsproteins ausgewählt ist.

5. Verfahren nach irgendeinem der Ansprüche 1 bis 4, wobei die Viskosität der Flüssigzusammensetzung um mindestens 12 %, vorzugsweise um mindestens 50 % reduziert wird.

6. Verfahren nach irgendeinem der Ansprüche 1 bis 5, wobei die Konzentration des Proteins zwischen 90 mg/ml und 250 mg/ml liegt.

7. Verfahren nach irgendeinem der Ansprüche 1 bis 6, wobei die Konzentration an Camphersulfonsäure weniger als etwa 500 mM, insbesondere weniger als 200 mM beträgt.

8. Verfahren nach irgendeinem der Ansprüche 1 bis 7, wobei der pH der Flüssigzusammensetzung im Bereich zwischen etwa 3 und etwa 8 liegt und einen Puffer enthält.

9. Flüssigzusammensetzung erhältlich durch das Verfahren irgendeines der Ansprüche 1 bis 8, die verglichen mit einer identischen Flüssigzusammensetzung ohne die Hilfsstoffkombinationen eine reduzierter Viskosität aufweist.

10. Verwendung des Verfahrens nach irgendeinem der Ansprüche 1 bis 8 in einem Bioprozess.

11. Verwendung des Verfahrens nach Anspruch 10, wobei der Permeatfluss der Flüssigzusammensetzung in einem Filtrationsschritt verglichen mit einer identischen Flüssigzusammensetzung, die Camphersulfonsäure und mindestens einen aus einer Gruppe bestehend aus Meglumin, Ornithin und Carnitin ausgewählten kationischen Hilfsstoff nicht enthält, erhöht ist.

12. Verwendung des Verfahrens nach den Ansprüchen 10 oder 11, wobei die Proteinrückgewinnung nach Pufferaustausch und Volumenreduzierung in einem Filtrationsschritt verglichen mit einer identischen Flüssigzusammensetzung, die Camphersulfonsäure und mindestens einen aus einer Gruppe bestehend aus Meglumin, Ornithin und Carnitin ausgewählten kationischen Hilfsstoff nicht enthält, erhöht ist.

## Revendications

1. Méthode de réduction de la viscosité d'une composition liquide comprenant une protéine en une concentration dans la plage allant d'au moins 50 mg/ml jusqu'à 300 mg/ml, comprenant l'étape consistant à combiner la composition liquide avec de l'acide camphosulfonique et au moins un excipient cationique, où le au moins un excipient cationique est choisi dans la liste comprenant la méglumine, l'ornithine et la carnitine selon une concentration ayant un effet réducteur de viscosité.

2. Méthode selon la revendication 1, dans laquelle la viscosité est réduite par rapport à une composition liquide identique ne comprenant ni acide camphosulfonique, ni au moins un excipient cationique choisi dans le groupe constitué par la méglumine, l'ornithine et la carnitine.

3. Méthode selon la revendication 1 ou 2, dans laquelle la composition liquide est une formulation pharmaceutique liquide, et la protéine est une protéine pharmaceutiquement active.

4. Méthode selon l'une quelconque des revendications 1 à 3, dans laquelle la protéine est choisie dans le groupe constitué par un anticorps, un fragment d'anticorps, un minicorps, un nanocorps, un anticorps modifié, une molécule de type anticorps, un conjugué anticorps-médicament et une protéine de fusion.

5. Méthode selon l'une quelconque des revendications 1 à 4, dans laquelle la viscosité de la composition liquide est réduite d'au moins 12%, préférablement d'au moins 50%.

6. Méthode selon l'une quelconque des revendications 1 à 5, dans laquelle la concentration de la protéine va de 90 mg/ml à 250 mg/ml.

7. Méthode selon l'une quelconque des revendications 1 à 6, dans laquelle la concentration en acide camphosulfonique est inférieure à environ 500 mM, notamment inférieure à 200 mM.

8. Méthode selon l'une quelconque des revendications 1 à 7, dans laquelle le pH de la composition liquide se trouve dans la plage allant d'environ 3 à environ 8 et comprend un tampon.

9. Composition liquide pouvant être obtenue par la méthode selon l'une quelconque des revendications 1 à 8, présentant une viscosité réduite par rapport à une composition liquide identique exempte des combinaisons d'excipient.

10. Utilisation de la méthode selon l'une quelconque des revendications 1 à 8 dans un bioprocédé.

11. Utilisation de la méthode selon la revendication 10, dans laquelle le flux de perméat de la composition liquide dans une étape de filtration est augmenté par rapport à une composition liquide identique ne comprenant ni acide camphosulfonique, ni au moins un excipient cationique choisi dans le groupe constitué par la méglumine, l'ornithine et la carnitine.

12. Utilisation de la méthode selon les revendications 10 ou 11, dans laquelle la récupération de protéine après l'échange de tampon et la réduction de volume dans une étape de filtration est augmenté par rapport à une composition liquide identique ne comprenant ni acide camphosulfonique, ni au moins un excipient cationique choisi dans le groupe constitué par la méglumine, l'ornithine et la carnitine.
